# EUROPEAN PATENT APPLICATION

(11) **EP 1 484 332 A2**
(43) Date of publication of application: **08.12.2004**
(21) Application number: 04015791.9
(22) Date of filing: 09.02.2001
(51) Int. Cl.: C07F 9/10

(54) **Lipid-based drug delivery system for administration of a drug substance**

(30) Priority: 10.02.2000 DK 200000211; 12.04.2000 DK 200000616
(62) Divisional of application: 01903609.4
(71) Applicant: Liplasome Pharma A/S, 2800 Lyngby (DK)
(72) Inventor: Jorgensen, Kent, DK-2880 Bagsvaerd (DK); Davidsen, Jesper, DK-2100 Copenhagen (DK); Vermehren, Charlotte, DK-2840 Holte (DK); Frokjaer, Sven, DK-2840 Holte (DK); Mouritsen, Ole G, DK-5230 Odense (DK)
(74) Representative: Englev, Peter

(57) **Abstract**

The present invention relates to a lipid based drug delivery system for administration of a drug substance, wherein the drug substance is incorporated in the system, said system including lipid derivatives which has (a) an aliphatic group of a length of at least 7 carbon atoms and an organic radical having at least 7 carbon atoms, and (b) a hydrophilic moiety, where the lipid derivative furthermore is a substrate for extracellular phospholipase A2 to the extent that the organic radical can be hydrolytically cleaved off, said system having included therein lipopolymers or glycolipids so as to present hydrophilic chains on the surface of the system.

## Description

### FIELD OF THE INVENTION

The invention relates to lipid-based pharmaceutical compositions used in the treatment of various disorders, e.g. cancer, infectious, and inflammatory conditions, etc., i.e. disorders and diseases associated with or resulting from increased levels of extracellular PLA₂ activity in the diseased tissue.

### BACKGROUND OF THE INVENTION

Mono-ether lyso-phospholipids and alkyl phosphocholines are known to be effective anticancer agents (see e.g. US 3,752,886 and later references). One specific example of a well-studied mono-ether alkyl phosphocholine is 1-O-octadecyl-2-O-methyl-*sn*-glycero-3-phosphocholine (EP 18-OCH₃).

Several mechanisms of the toxic action of ether-lipids towards cancer cells have been proposed involving lack of alkyl-cleavage enzymes in cancer cells. This can leads to an accumulation of the ether-lipids in the cell membranes which induce membrane defects and possibly subsequent lysis. Other potential mechanisms of action include effects on intracellular protein phosphorylation and disruption of the lipid metabolism. Normal cells typically possess alkyl-cleavage enzymes, which enable them to avoid the toxic effect of ether-lipids. However, some normal cells e.g., red blood cells, have like cancer cells no means of avoiding the disruptive effect of the etherlipids. Accordingly, therapeutic use of ether-lipids requires an effective drug-delivery system that protects the normal cells from the toxic effects and is able to bring the etherlipid to the diseased tissue.

Lohmeyer and Workman, Brit. J. Cancer, describe the cytotoxic effect of arachidonoyl-PAF₁₆ *in vitro.*

US 5,827,836 discloses retinoyl substituted glycerophophoethanolamines. It is stated that the compounds and salts thereof exhibit antitumor, anti-psoriatic and anti-inflammatory activities. A possible class of compounds has a fatty ether substituent in the 1-position, a retinoid ester (retinoyl) substituent in the 2-position and a phosphoethanolamine substituent in the 3-position. It is mentioned that some of the compounds can be presented in a liposome formulation.

US 4,372,949 discloses a carcinostatic and immunostimulating agent containing a lysophospholipid and a phospholipid. Examples of compounds are 3-phosphorylcholine having a C₅₋₂₂-acyloxy or C₅₋₂₂-alkoxy substituent in the 1-position, and a hydrogen, hydroxy, C₁₋₅-acyloxy or C₁₋₅-alkoxy substituent in the 2-position. It is mentioned that the agents can be dispersed in the form of micelles or lipid vesicles.

US 5,484,911 discloses nucleoside 5'-diphosphate conjugates of ether lipids which exhibit antiviral activity. The compounds may have a fatty ether/thioether substituent in the *sn*-1-position and a fatty acid ester substituent in the *sn*-2-position. The compounds are designed so as to penetrate the cell membrane whereafter the nucleoside drug is liberated by cleavage by intracellular phosphatases. It is furthermore suggested that the also liberated ether lipids may be subsequently cleaved by intracelluar phospholipase A₂. It is suggested that the conjugates can be presented in the form of micelles which more easily can be taken up by macrophages.

US 4,622,392 discloses cytotoxic compounds of the nucleotide-lipid conjugate type.

ES 2 034 884 discloses 2-aza-phospholipider as PLA₂ inhibitors. Similarly, de Haas et al (Biochem. Biophys. Acta, Lipid and Lipids Metabolism, 1167 (1993) No. 3, pp 281-288, discloses inhibition of pancreatic PLA₂ by (R)-2-acylamino phospholipid analogues.

Hoffman et al., Blood, Vol. 63, No. 3 (March), 1984, pp 545-552, discloses the cytotoxicity of PAF and related alkyl-phospholipid analogues in human Leukemia cells.

WO 94/09014 discloses phosphoric acid esters as PLA₂ inhibitors. A group of the inhibitors are 1-O-phospho-2-O-(C₂₋₂₁-acyl)-(C₂₋₂₄-alkanes).

Xia and Hui discloses the chemical synthesis of a series of ether phospholipids from D-mannitol and their properties as tumor-cytotoxic agents.

US 5,985,854, US 6,077,837, US 6,136,796 and US 6,166,089 describe prodrugs with enhanced penetration into cells, which are particular useful for treating a condition or disease in a human related to supranormal intracellular enzyme activity. The prodrugs may be *sn*-2-esters of lysophospholipids. Such drugs are designed so as to be cleaved by intracelluar phospholipase A₂.

Even in view of the above, an increasing demand for novel drug delivery systems exist, in particular drug delivery systems for targeted delivery of drug substances which are able to treat or alleviate conditions such as cancer and inflammation. Due to the fact that drugs for the treatment of cancer may be particularly harmful to tissue in general, it is of particular importance to suppress liberation of the drug substance or substances at locations other than the diseased tissue.

### BRIEF DESCRIPTION OF THE INVENTION

The present invention is directed to drug delivery systems which are particularly useful in the treatment or alleviation of diseases which are characterised by localised activity of extracelluar PLA₂ activity.

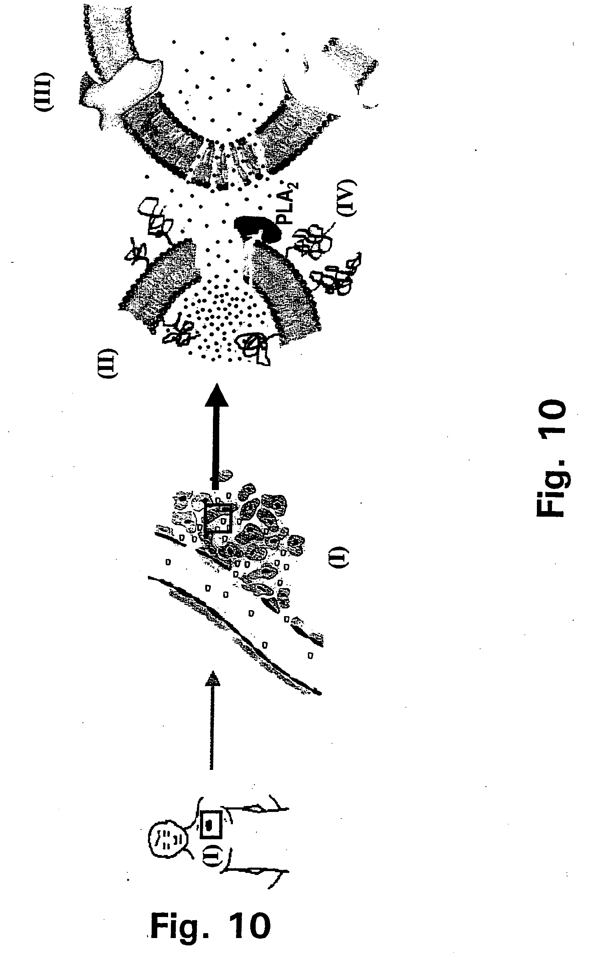

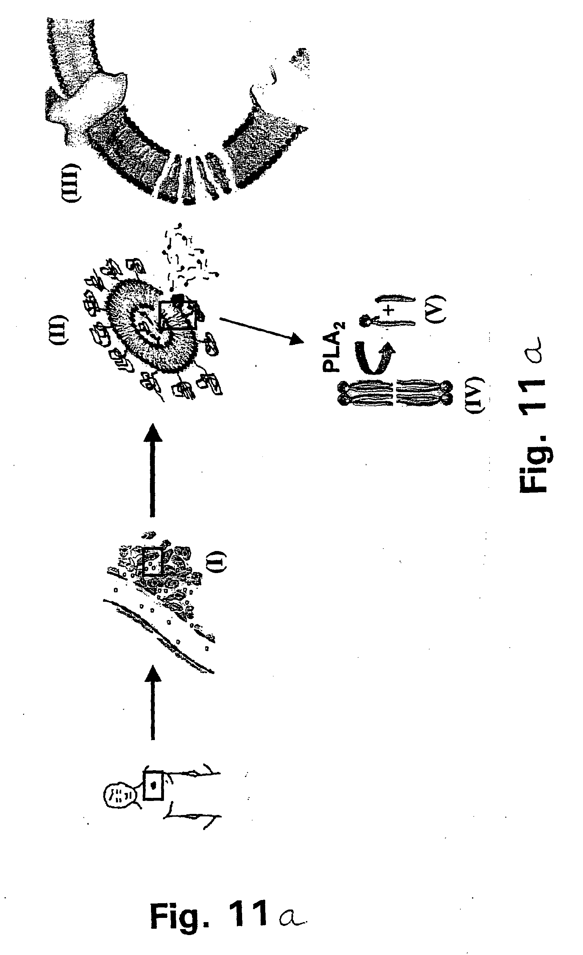

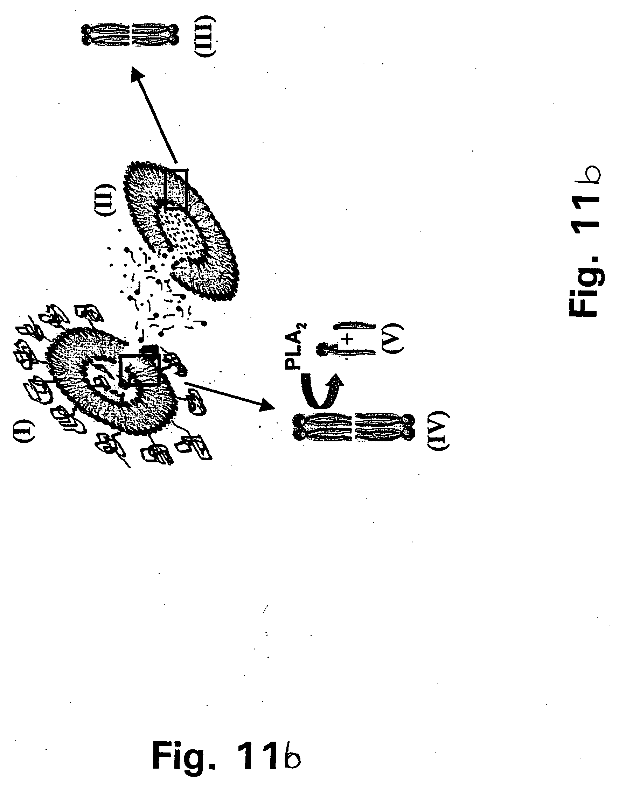

The new principle for liposomal drug targeting by extracellular PLA₂ described in this application - involves lipid-based prodrugs as illustrated in Figs. 10 and 11. In this case a specific lipid-analogue compound may be incorporated into the polymer or polysaccharide chains "grafted" carrier liposome and act as a prodrug which is turned into an active drug by hydrolysis via the extracellular phospholipase. Possible examples could be certain mono-ether lipids which have been found to exhibit anti-cancer activity. If the mono-ether lipids are modified with a long fatty-acid chain that is ester linked in the sn-2-position and therefore can be hydrolysed by extracellular PLA₂ at the target site, these modified mono-ether lipids constituting the carrier liposome will act as prodrugs. Finally it should be pointed out that certain drugs such as the anti-cancer drug adriamycin (of which doxorubicin is a derivative) themselves are known to stimulate extracellular PLA₂-activity by decreasing the calcium requirement of the enzyme.

The principle of drug targeting, release and absorption by extracellular phospholipase A2 (PLA₂) which is illustrated in Figs. 10 and 11, can be applied to a case also involving lipid-based prodrugs. In this case lipid derivatives are constituents of the carrier liposome and act as prodrugs which are turned into active drugs (e.g. ether lipids) by hydrolysis via the extracellular PLA₂ that is present in elevated concentrations in the diseased target tissue.

A specific example is a prodrug of a certain mono-ether lipid which exhibits anti-cancer activity. This can be a therapeutically active compound (e.g. regulatory fatty acid derivatives) that is ester bound to the phospholipid in the sn-2 position and therefore renders the lipid derivative substrate for extracellular PLA₂. If the mono-ether lipids are modified with, e.g. a ester-linked derivative in the sn-2-position and therefore can be hydrolysed by extracellular PLA₂ at the target site, these lipid derivatives constituting the carrier liposome will act as prodrugs that become hydrolysed and turned into drugs by extracellular PLA₂ at the target site. In this way therapeutically active substances, e.g., monoether lipids and ester-linked derivatives will be liberated at the desired target site. Furthermore, the hydrolysis product can act as local permeability enhancers facilitating the transport of the generated anti-cancer drug into the cell. Pharmaceutical compositions containing the lipid-based system can be used therapeutically, for example, in the treatment of cancer, infectious and inflammatory conditions.

This invention provides such a delivery system in the form of lipid-based carriers, e.g. liposomes or micelles, composed of lipid-bilayer forming ether-lipids such as glycerophospholipids containing an alkyl-linkage in the 1-position and an acyl-linkage in the sn-2-position on the glycerol backbone and which have polymer or polysaccharide chains grafted thereto. In addition, the carrier system may contain lipid-bilayer stabilising components, e.g. lipopolymers, glycolipids and sterols which lead to an increased vascular circulation time and as a consequence an accumulation in the diseased target tissue. When the carriers reach the target site of therapeutic action, e.g. cancer cells, PLA₂-catalyzed hydrolysis of the acyl-linkage releases the therapeutically active components, typically lyso-etherlipids and ester-linked derivatives. Contradictory to alkyl-cleavage enzymes which are nearly absent in cancer cells, extracellular PLA₂ activity is elevated in cancer tissue. In addition, extracellular PLA₂ activity is elevated in diseased regions such as inflammatory tissue.

The present invention thus provides a lipid-based drug delivery system for administration of an active drug substance selected from lysolipid derivatives, wherein the active drug substance is present in the lipid-based system in the form of a prodrug, said prodrug being a lipid derivative having (a) an aliphatic group of a length of at least 7 carbon atoms and an organic radical having at least 7 carbon atoms, and (b) a hydrophilic moiety, said prodrug furthermore being a substrate for extracellular phospholipase A2 to the extent that the organic radical can be hydrolytically cleaved off, whereas the aliphatic group remains substantially unaffected, whereby the active drug substance is liberated in the form of a lysolipid derivative which is not a substrate for lysophospholipase, said system having included therein lipopolymers or glycolipids so as to present hydrophilic chains on the surface of the system.

The present invention also provides a lipid based drug delivery system for administration of an second drug substance, wherein the second drug substance is incorporated in the system, said system including lipid derivatives which has (a) an aliphatic group of a length of at least 7 carbon atoms and an organic radical having at least 7 carbon atoms, and (b) a hydrophilic moiety, where the lipid derivative furthermore is a substrate for extracellular phospholipase A2 to the extent that the organic radical can be hydrolytically cleaved off, whereas the aliphatic group remains substantially unaffected, so as to result in an organic acid fragment or an organic alcohol fragment and a lysolipid fragment, said lysolipid fragment not being a substrate for lysophospholipase, said system having included therein lipopolymers or glycolipids so as to present hydrophilic chains on the surface of the system.

Thus, the present invention takes advantage of the surprising finding that liposomes (and micelles) including lipid derivatives which can be specifically and only partially cleaved by extracellular phospholipases, and which at the same time includes lipopolymers or glycolipids, have the properties of circulating in the blood stream sufficiently long so as to reach target tissue where the extracellular PLA₂ activity is elevated without being recognised by the mammalian reticuloendothelial systems and without penetrating cell walls, whereby the lipid derivatives of the liposomes are specifically cleaved by extracellular PLA₂ so as to liberate therapeutically active ingredients at the desired location.

The present invention also provides a class of novel lipid derivatives which are particularly useful as constituents of the drug delivery systems described herein.

### DESCRIPTION OF THE DRAWINGS

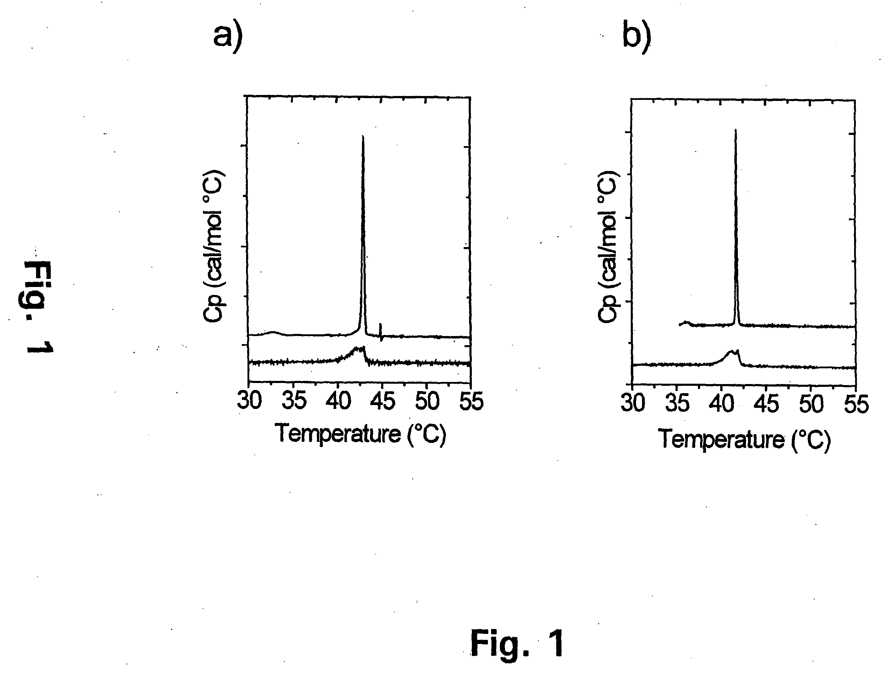

Fig. 1. Heat capacity curves obtained using differential scanning calorimetry. (a) Multilamellar, MLV (the upper curve) and unilamellar, LUV (the bottom curve) liposomes made of 1 mM 1-O-hexadecyl-2-hexadecanoyl-*sn*-glycero-3-phosphocholine (1-O-DPPC). (b) MLV (the upper curve) and LUV (the bottom curve) liposomes made of dipalmitoylphosphatidylcholine (DPPC).

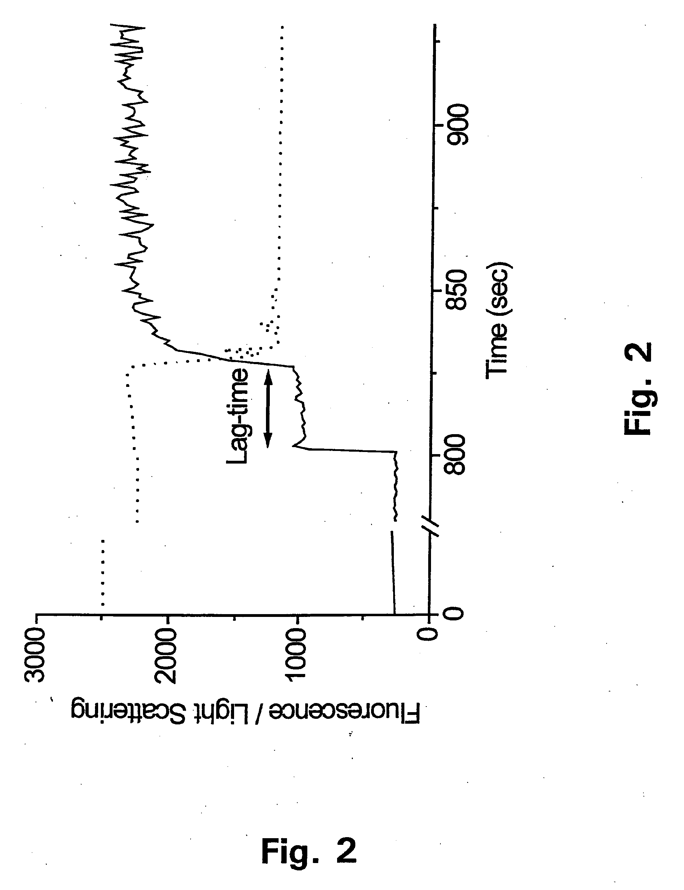

Fig. 2. Characteristic reaction time profile at 41°C for phospholipase A₂, PLA₂, (A. piscivorus piscivorus) hydrolysis of unilamellar 1-O-DPPC-liposomes composed of 90% 1-O-DPPC and 10% 1-O-hexadecyl-2-hexadecanoyl-*sn*-glycero-3-phosphoethanolamine-N-[methoxy(polyethylene glycol)-350] (1-O-DPPE-PEG350). The PLA₂ hydrolysis reaction is monitored by intrinsic fluorescence (solid line) from the enzyme and 90° static light scattering (dashed lines) from the lipid suspension. After adding PLA₂, at 800 sec to the equilibrated liposome suspension a characteristic lag-time follows before a sudden increase in the catalytic activity takes place. Samples for HPLC were taken before adding the enzyme and 20 minutes after the observed lag time.

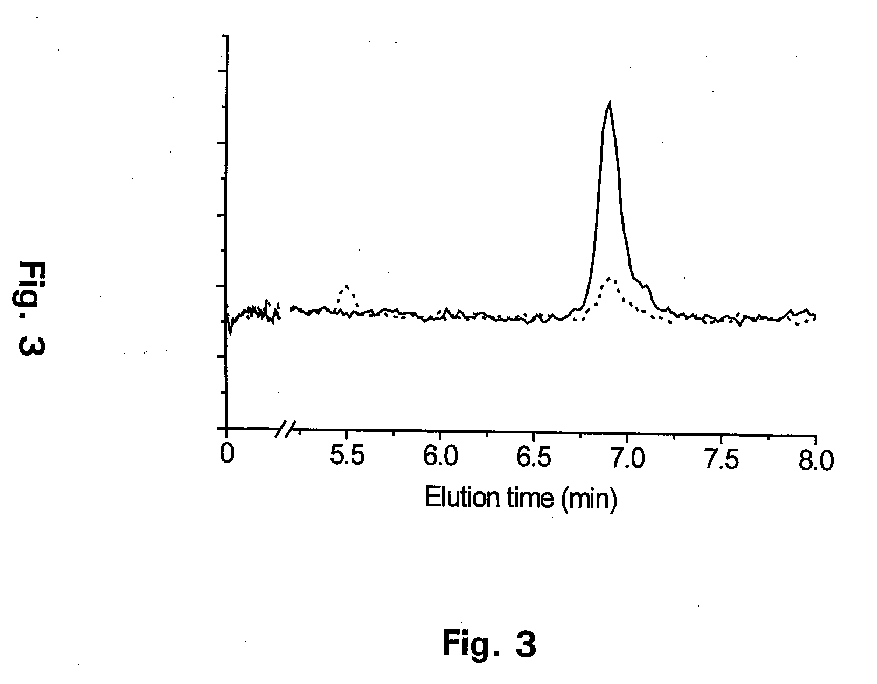

Fig. 3. HPLC chromatograms illustrating the effect of phospholipase A₂ hydrolysis of liposomes composed of 90% 1-O-DPPC and 10% 1-O-DPPE-PEG350. The chromatograms show the amount of 1-O-DPPC (100%, solid line) before phospholipase A₂ (A. piscivorus piscivorus) was added to the liposome suspension and the amount of 1-O-DPPC (75%, dashed line) after the lag-burst.

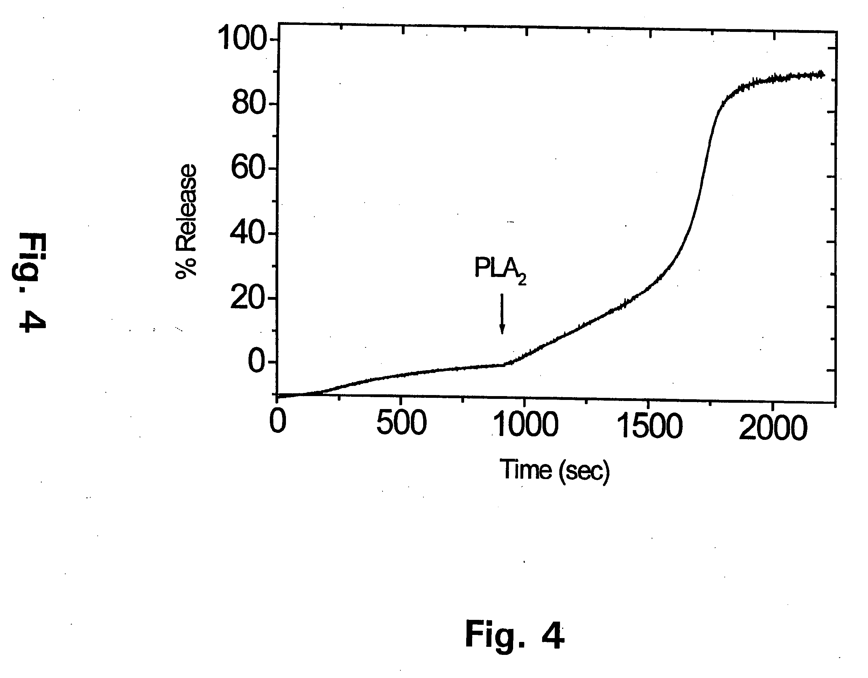

Fig. 4. PLA₂-controlled release of the fluorescent model drug calcein from liposomes composed of 25 µM 1-O-hexadecyl-2-hexadecanoyl-*sn*-glycero-3-phosphocholine (1-O-DPPC) suspended in a 10 mM HEPES-buffer (pH = 7.5), as a function of time. 25 nM phospholipase A₂ (A. piscivorus piscivorus) was added at time 900 sec, the temperature was 37°C. The percentage of calcein released is determined as % Release =100 (I_{F(t)}-I_{B})/(I_{T} - I_{B}), where I_{F(t)} is the measured fluorescence at time t after addition of the enzyme, I_{B} is the background fluorescence, and I_{T} is the total fluorescence measured after addition of Triton X-1 00 which leads to complete release of calcein by breaking up the liposomes.

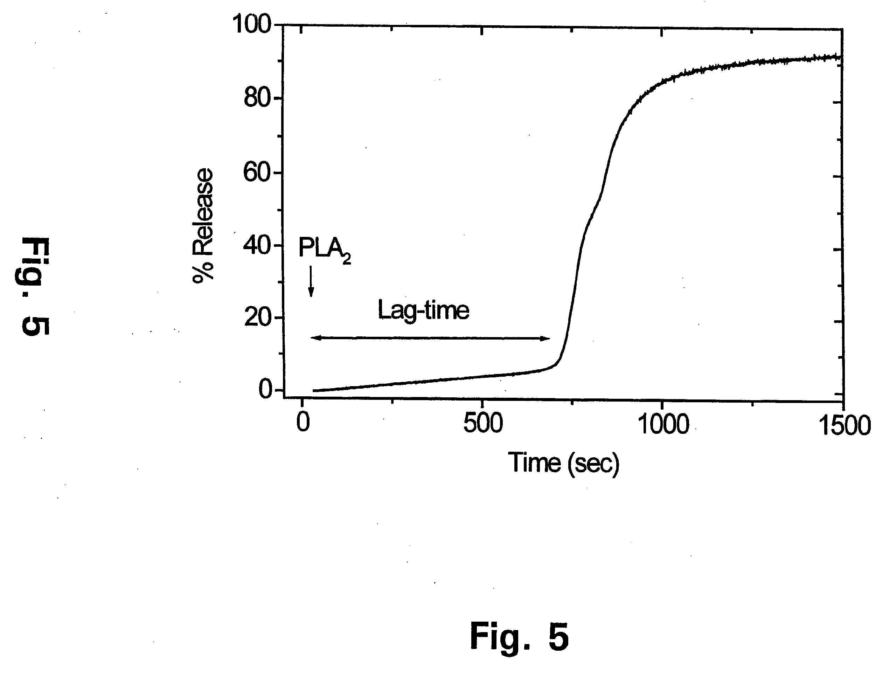

Fig. 5. PLA₂-controlled release of the fluorescent model drug calcein across the target membrane of non-hydrolysable membranes (see Fig. 11b), as a function of time for liposomes composed of 25 µM 1-O-hexadecyl-2-hexadecanoyl-*sn*-glycero-3-phosphocholine (1-O-DPPC) suspended in a 10 mM HEPES-buffer (pH = 7.5). 25 nM phospholipase A₂ was added at time 0 sec and the temperature was 37°C. The percentage of calcein released is determined as described in Fig. 4.

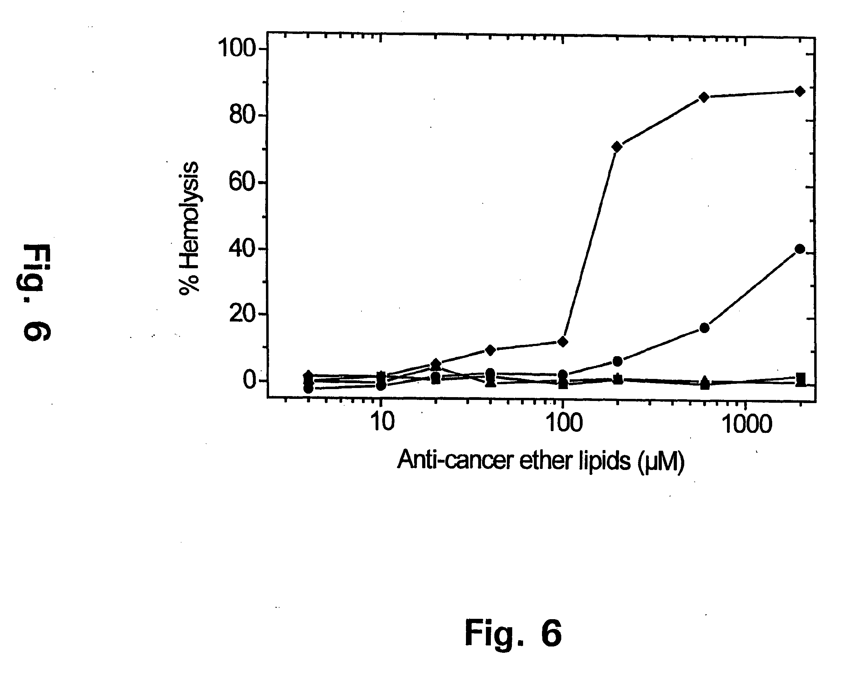

Fig. 6. Hemolysis profile of normal red blood cells in the presence of liposomes composed of 100% 1-O-DPPC (squares); 90% 1-O-DPPC and 10% 1-O-DPPE-PEG350 (triangles); 90% 1-O-DPPC and 10% 1-O-DPPE-PEG2000 (circles) and ET-18-OCH₃ (diamonds). The concentrations that yield 5% hemolysis (H₅) were well above 2 mM for liposomes composed of 100% 1-O-DPPC, and for liposomes composed of 90%1-O-DPPC with 10% DPPE-PEG350. Hemolysis assay was performed as described by Perkins et al., 1997, Biochimica et Biophysica Acta 1327, 61-68. Briefly, each sample was serially diluted with phosphate buffered saline (PBS), and 0.5 ml of each dilute suspension was mixed with 0.5 ml washed human red blood cells (RBC) [4% in PBS (v/v)]. Sample and standard were placed in a 37°C incubator and agitated for 20 hours. Tubes were centrifuged at low speed (2000 x G) for 10 minutes and 200 µl of the supernatant was quantitated by absorbance at 550 nm. 100 percent hemolysis was defined as the maximum amount of hemolysis obtained from the detergent Triton X-100. The hemolysis profile in Fig. 6 shows a low hemolysis value (below 5% percent) for 2 mM 1-O-DPPC-liposomes and for 1-O-DPPC with 10% 1-O-DPPE-PEG350, liposomes.

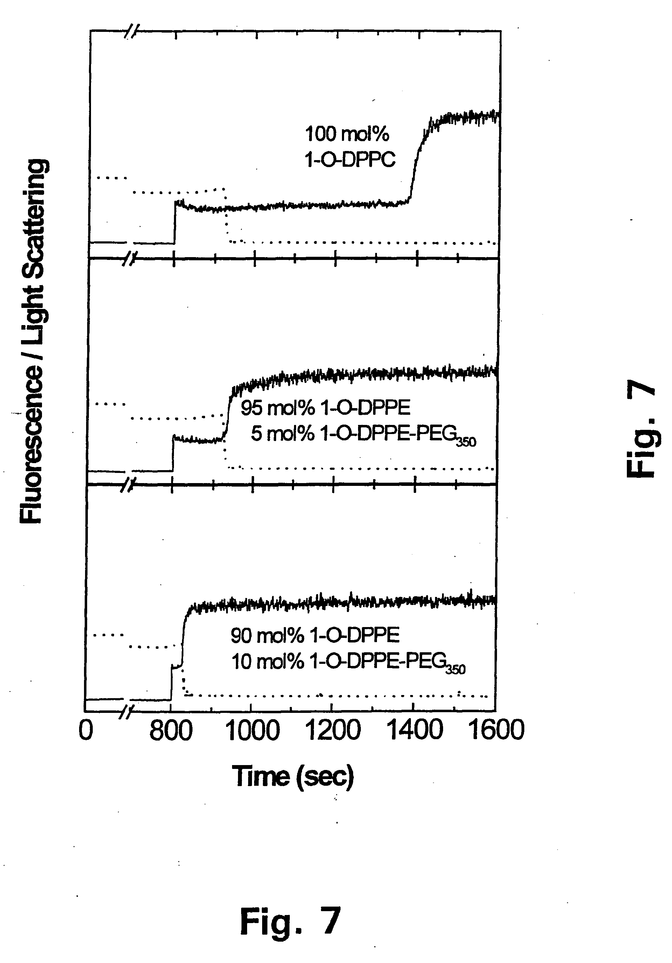

Fig. 7. Characteristic reaction time profiles at 41 °C for PLA₂ (A. piscivorus piscivorus) hydrolysis of unilamellar liposomes incorporated with 0, 5 and 10% 1-O-DPPE-PEG350 lipopolymers. The PLA₂ hydrolysis reaction is monitored by intrinsic fluorescence (solid line) from the enzyme and 90° static light scattering (dashed lines) from the suspension. After adding PLA₂ to the equilibrated liposome suspension a characteristic lag-time follows before a sudden increase in the catalytic activity takes place.

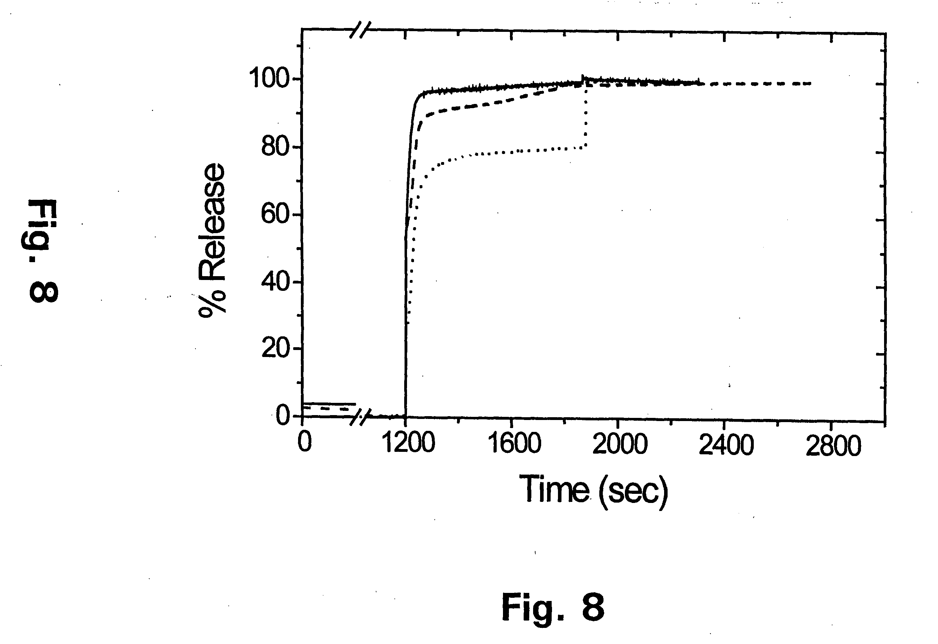

Fig. 8. PLA₂-controlled release of the fluorescent model drug calcein across the target membrane of non-hydrolysable membranes as a function of time for micelles composed of 25 µM 1-O-DPPE-PEG350 (dotted line), DSPE-PEG750 (dashed line), 1-O-DPPE-PEG2000 (solid line) suspended in a 10 mM HEPES-buffer (pH = 7.5). Phospholipase A₂ (25 nM) was added at time 1200 sec and the temperature was 41°C. The percentage of calcein released is determined as described in Fig. 4. PLA₂ catalysed hydrolysis of 1-O-DPPE-PEG350 induced the fastest and highest release.

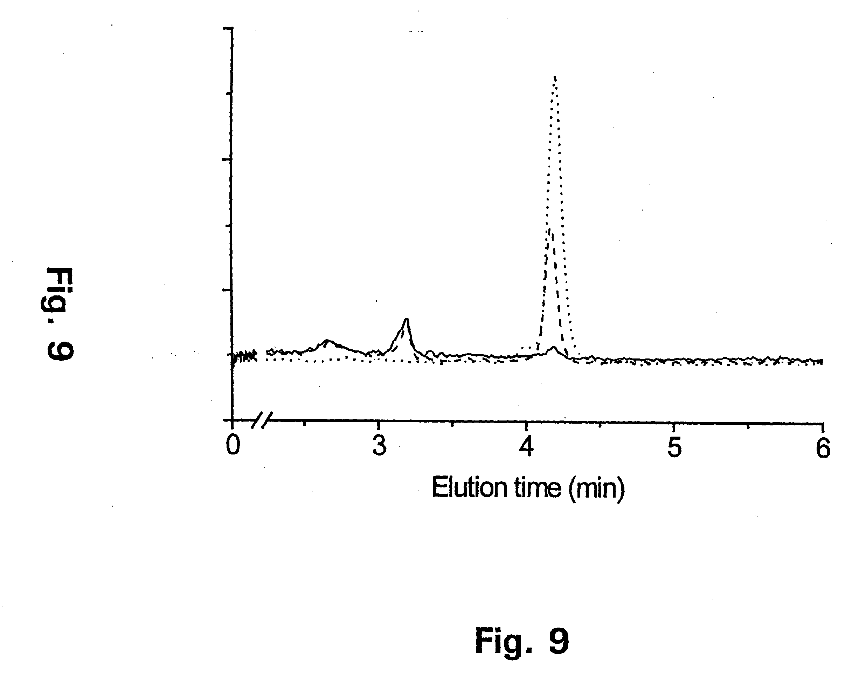

Fig. 9. HPLC chromatograms illustrating the effect of phospholipase A₂ hydrolysis of micelles composed DSPE-PEG750 (0.150 mM). The chromatograms show the amount of stearic acid generated before (solid line) phospholipase A₂ (A. piscivorus piscivorus) was added to the micelle suspension and the amount (dashed line) of DSPE-PEG750 after the lag-burst. The dottet lineshows pure stearic acid (0.4 mM). The percentage hydrolysis was calculated on basis of the integrated area of the stearic acid standard (115850 units) and the integrated area of the sample (45630 units). The concentration of the stearic acid in the sample was calculated to (45630/115850 x 0.4 mM) 0.157 mM, which means that 100% of the DSPE-PEG750 was hydrolysed to lyso-DSPE-PEG750 and stearic acid.

Fig. 10. Describes the principle of liposomal drug targeting, release and absorption by extracellular enzymes.
(I) Pathological tissue with leaky capillaries
(II) Liposomal drug carrier
(III) Target cell and cell membrane
(IV) Localised drug release and absorption by extracellular phospholipase A2

Fig.11 (a). Schematic illustration of a liposomal drug-targeting principle involving accumulation of the liposomal drug carriers in porous diseased tissue and subsequent release of drug and transport across the target membrane via extracellular PLA₂ activity.
(I) Pathological tissue with leaky capillaries
(II) Polymer-stabilised prodrug liposome
(III) Target cell and cell membrane
(IV) Prodrug (monoether-lipid), proenhancer (lipid), proactivator (lipid)
(V) Drugs (ether-lysolipid and fatty acid derivatives), enhancers (lysolipid + fatty acid), PLA₂ activators (lysolipid + fatty acid)

(b) Schematic illustration of a molecular-based biophysical model system where the phospholipids of the carrier liposomes, via the PLA₂-catalysed hydrolysis, act as prodestabilisers at the site of the carrier and as proenhancers at the site of the target. The possibility of extending the principle to include a lipid-based prodrug is also included.
(I) Polymer stabilised carrier liposome
(II) Non-degradable target liposomal membrane
(III) Non-hydrolysable ether-lipids
(IV) Proenhancer (lipid), prodrug (monoether-lipid), proactivator (lipid)
(V) Enhancers (lysolipid + fatty acid), drugs (ether-lysolipid and fatty acid derivatives), PLA₂ activators (lysolipid + fatty acid)

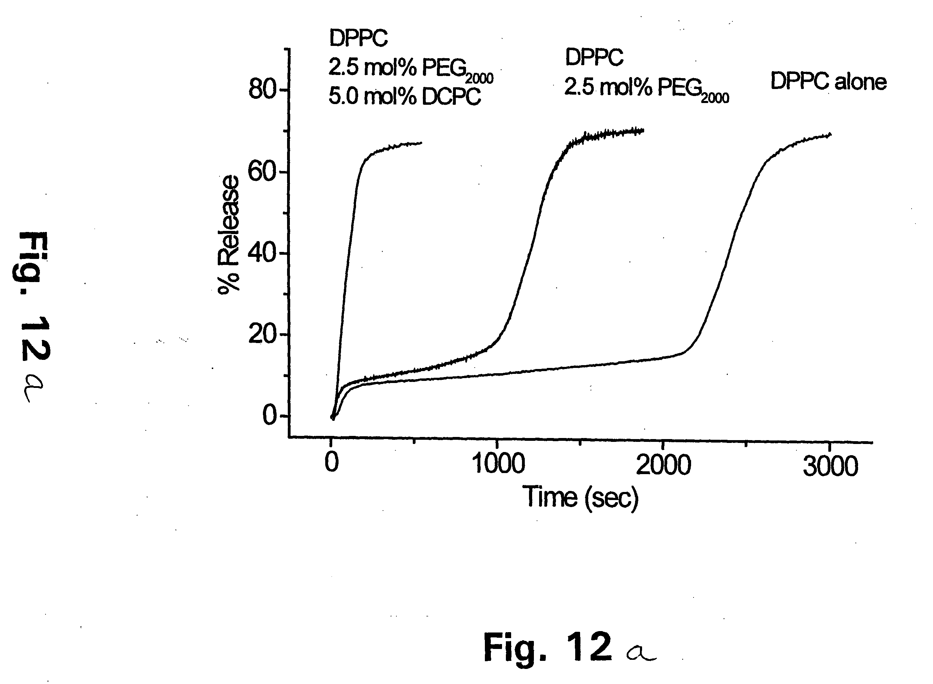

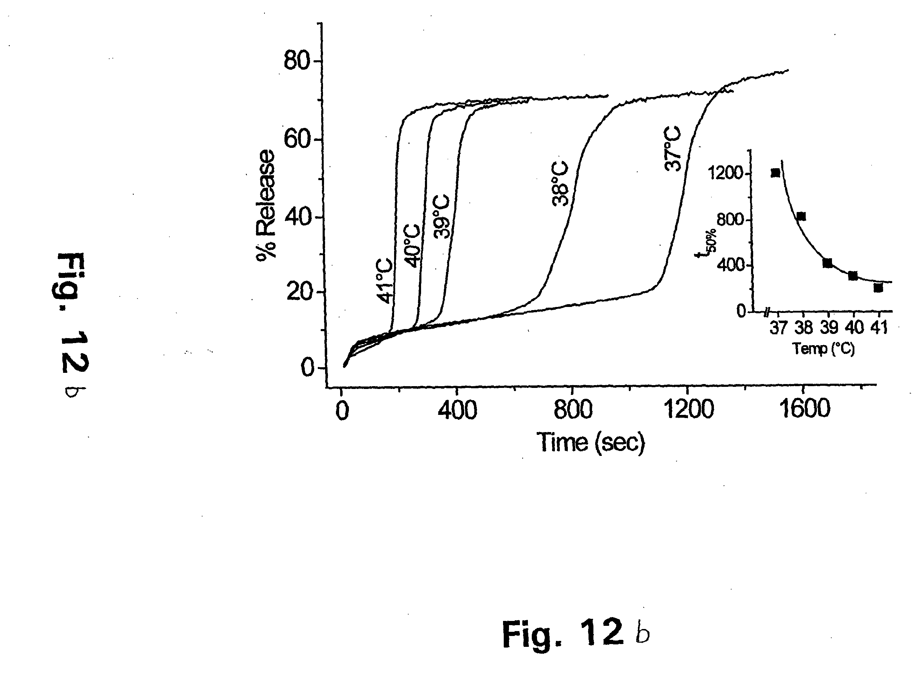

Fig. 12 (a) PLA₂-controlled release of the fluorescent model drug calcein across the target membrane as a function of time for different compositions of the carrier liposomes. The temperature is 37°C. In comparison with bare DPPC carriers, the rate of release of the model drug is dramatically enhanced for the polymer-coated carriers, DPPC + 2.5 mol% DPPE-PEG2000. A further augmentation of the rate of release is obtained if the carrier also contains a short-chain phospholipid, DCPC, which acts as a local activator for the enzyme. The percentage of calcein released is determined as
% Release =100 (I_{F(t)}-I_{B})/(I_{T} - I_{B}), where I_{F(t)} is the measured fluorescence at time t after addition of the enzyme, I_{B} is the background fluorescence, and I_{T} is the total fluorescence measured after addition of Triton X-1 00 which leads to complete release of calcein by breaking up the target liposomes. (b) PLA₂-controlled release of the fluorescent model drug calcein across the target membrane as a function of time for different temperatures. As the temperature is raised, the rate of release is enhanced due to increased activity of the enzyme induced by structural changes in the lipid bilayer substrate of the carrier liposome. In the present assay a maximum release of about 70% is achieved in all cases. The insert shows the time of 50% calcein release, t_{50%}, as a function of temperature. The concentration of the target and carrier liposomes are 25 µM, and PLA₂ is added in a 25 nM concentration in a HEPES buffer with pH=7.5.

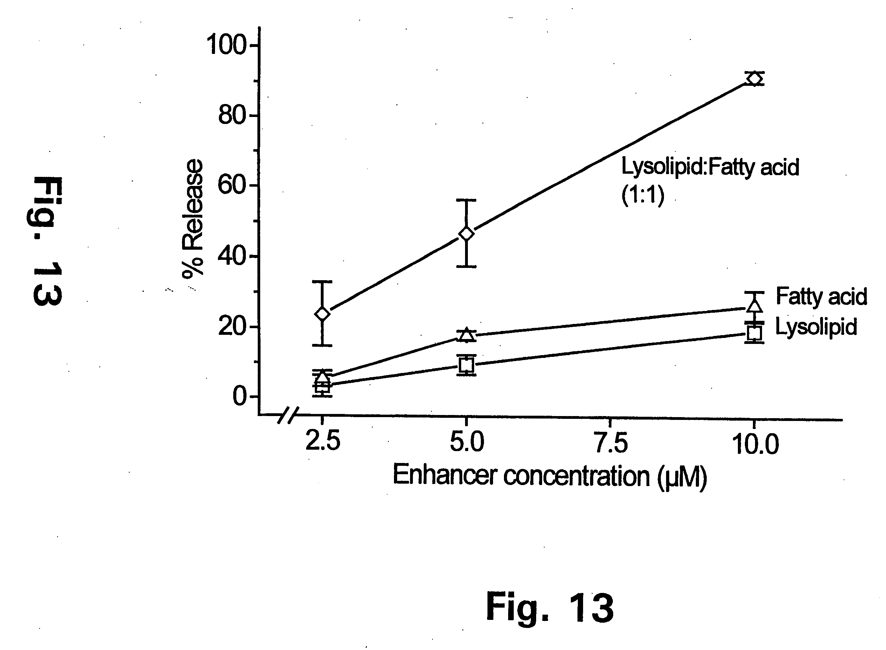

Fig. 13. Total release after 20 min of the fluorescent model drug calcein across the target membrane as a function of adding increasing amounts of lyso-palmitoyl phospholipid and palmitic acid, separately, and in a 1:1 mixture. The concentration of the target membranes is 25 µM in a HEPES buffer with pH=7.5 at a temperature of 39°C.

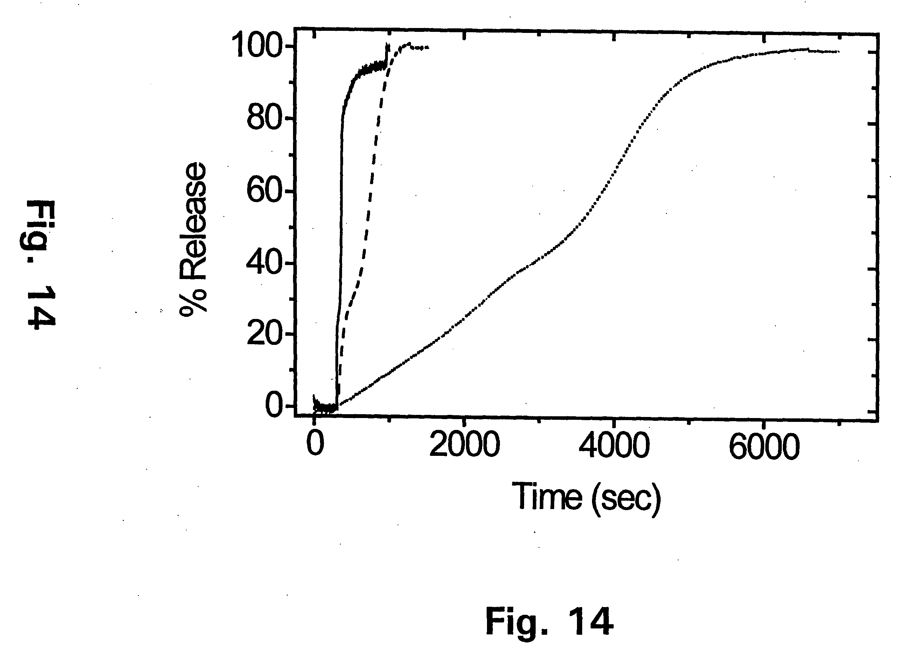

Fig 14. PLA₂-controlled release of the fluorescent model drug calcein from liposomes composed of 25 µM 90 mol% 1-O-hexadecyl-2-hexadecanoyl-*sn*-glycero-3-phosphocholine (1-O-DPPC) and 10 mol% 1-O-hexadecyl-2-hexadecanoyl-*sn*-glycero-3-phosphoethanolamine-N-[methoxy(polyethylene glycol)-350] (1-O-DPPE-PEG350) suspended in a 10 mM HEPES-buffer (pH = 7.5), as a function of time. 50 nM (straight line), 1 nM (solid line) and 0.02 nM (dotted line) phospholipase A₂ (A. piscivorus piscivorus) was added at time 300 sec, the temperature was 35.5°C. The percentage of calcein released is determined as describe in Fig 4.

### DETAILED DESCRIPTION OF THE INVENTION

One of the important features of the present invention is the realisation that certain lipid derivatives will be cleaved by extracellular PLA₂ in a well-defined manner in extracellular locations of mammalian tissue. It has been found that extracellular PLA₂ is capable of cleaving monoether/monoester lipid derivatives so as to produce monoether lysolipid derivatives which as such, or in combination with other active compounds, will exhibit a therapeutic effect.

### Lipid derivatives

Thus, the drug delivery systems (liposomes or micelles) of the present invention relies on lipid derivative having (a) an aliphatic group of a length of at least 7 carbon atoms and an organic radical having at least 7 carbon atoms, and (b) a hydrophilic moiety, said prodrug furthermore being a substrate for extracellular phospholipase A2 to the extent that the organic radical can be hydrolytically cleaved off, whereas the aliphatic group remains substantially unaffected, whereby the active drug substance is liberated in the form of a lysolipid derivative which is not a substrate for lysophospholipase, said system having included therein lipopolymers or glycolipids so as to present hydrophilic chains on the surface of the system.

Although the terms "lipid" and "lysolipid" (in the context of phospholipids) will be well-known terms for the person skilled in the art, it should be emphasised that, within the present description and claims, the term "lipid" is intended to mean triesters of glycerol of the following formula: 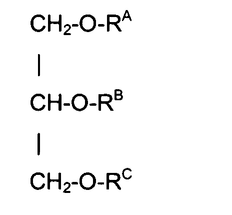 wherein R^{A} and R^{B} are fatty acid moieties (C₉₋₃₀-alkyl/alkylene/alkyldiene/alkyltriene/alkyltetraene-C(=O)-) and R^{C} is a phosphatidic acid (PO₂-OH) or a derivative of phosphatidic acid. Thus, the groups R^{A} and R^{B} are linked to the glycerol backbone via ester bonds.

The term "lysolipid" is intended to mean a lipid where the R^{B} fatty acid group is absent (e.g. hydrolytically cleaved off), i.e. a glycerol derivative of the formula above where R^{B} is hydrogen, but where the other substituents are substantially unaffected. Conversion of a lipid to a lysolipid can take place under the action of an enzyme, specifically under the action of celluar as well as extracellular PLA₂.

The terms "lipid derivative" and "lysolipid derivative" are intended to cover possible derivatives of the above possible compounds within the groups "lipid" and "lysolipid", respectively. Examples of biologically active lipid derivatives and lysolipid derivatives are given in Houlihan, et al., Med. Res. Rev., 15, 3, 157-223. Thus, as will be evident, the extension "derivative" should be understood in the broadest sense.

Within the present application, lipid derivatives and lysolipids should however fulfil certain functional criteria (see above) and/or structural requirements. It is particularly relevant to note that the suitable lipid derivatives are those which have (a) an aliphatic group of a length of at least 7, preferably at least 9, carbon atoms and an organic radical having at least 7 carbon atoms, and (b) a hydrophilic moiety. It will be evident that the aliphatic group and the organic radical will correspond to the two fatty acid moieties in a normal lipid and that the hydrophilic moiety will correspond to the phosphate part of a (phospho)lipid or a bioisoster thereof.

Thus, as the general idea behind the present invention is to exploit the increased level of extracellular PLA₂ activity in localised areas of the body of a mammal, in particular diseased tissue, the lipid derivatives which can be utilised within the present invention should be substrates for extracellular PLA₂, i.e. the lipid derivatives should be able to undergo hydrolytic, enzymatic cleavage of the organic radical corresponding to the fatty acid in the 2-position in a lipid. Extracellular PLA₂ is known to belong to the enzyme class (EC) 3.1.1.4. Thus by reference to (extracellular) PLA₂ should be understood all extracellular enzymes of this class, e.g. lipases, which can induce hydrolytic cleavage of the organic radical corresponding to the fatty acid in the 2-position in a lipid. One particular advantage of the lipid based drug delivery system (as liposomes and micelles) is that extracellular PLA₂ activity is significantly increased towards organised substrates as compared to monomeric substrates.

In view of the requirement to hydrolysability by extracellular PLA₂, it is clear that the organic radical (e.g. aliphatic group) is preferably linked via an ester functionality which can be cleaved by extracellular PLA₂, preferably so that the group which is cleaved off is a carboxylic acid.

Furthermore, it is an important feature of the present invention that the aliphatic group (the group corresponding to the fatty acid in the 1-position in a lipid) of the lipid derivative, i.e. the lysolipid derivative after cleavage by extracellular PLA₂, is substantially unaffected by the action of extracellular PLA₂. By "substantially unaffected" is meant that the integrity of the aliphatic group is preserved and that less than 1 mol%, preferably less than 0.1 mol%, of the aliphatic group (the aliphatic group in the 1-position) is is cleaved under the action of extracellular PLA₂.

Also, the lysolipid derivative resulting from the hydrolytic cleavage of the organic radical should not in itself be a substrate for lysophospholipase. Lysophospholipase is known to belong to the enzyme class (EC) 3.1.1.5. Thus by reference to lysophospholipase should be understood all enzymes of this class that catalyses the reaction lyso(phospho)lipid + water yielding phosphoglycerol + fatty acid. The term "not a substrate for lysophospholipase" is intented to mean that lysophospholipase has an activity of less than 1% towards the substrate compared with the corresponding esterlipid, i.e. virtually not enzymatic activity.

Suitable examples of such lysolipid derivatives are those which will not undergo hydrolytical cleavage under the action of lysophospholipases. Thus, the lysolipid derivatives are in particular not lysolipids and lysolipid derivatives which have an ester linkage in the 1-position of the lysolipid or the position of a lysolipid derivative which corresponding to the 1-position of a lysolipid.

One preferred class of lipid derivatives for incorporation in the drug delivery systems of the invention can be represented by the following formula: 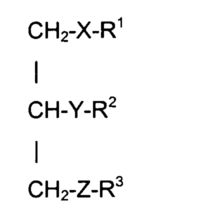 wherein
X and Z independently are selected from O, CH₂, NH, NMe, S, S(O), and S(O)₂, preferably from O, NH, NMe and CH₂, in particular O;
Y is -OC(O)-, Y then being connected to R² via either the oxygen or carbonyl carbon atom, preferably via the carbonyl carbon atom;
R¹ is an aliphatic group of the formula Y¹Y²;
R² is an organic radical having at least 7 carbon atoms, such as an aliphatic group having a length of at least 7, preferably at least 9, carbon atoms, preferably a group of the formula Y¹Y²;
where Y¹ is -(CH₂)ₙ₁-(CH=CH)ₙ₂-(CH₂)ₙ₃-(CH=CH)ₙ₄-(CH₂)ₙ₅-(CH=CH)ₙ₆-(CH₂)ₙ₇-(CH=CH)ₙ₈ᵣ-(CH₂)ₙ₉, and the sum of n1+2n2+n3+2n4+n5+2n6+n7+2n8+n9 is an integer of from 9 to 29; n1 is zero or an integer of from 1 to 29, n3 is zero or an integer of from 1 to 20, n5 is zero or an integer of from 1 to 17, n7 is zero or an integer of from 1 to 14, and n9 is zero or an integer of from 1 to 11; and each of n2, n4, n6 and n8 is independently zero or 1; and Y² is CH₃ or CO₂H; where each Y¹-Y² independently may be substituted with halogen or C₁₋₄-alkyl, but preferably Y¹-Y² is unsubstituted,
R³ is selected from phosphatidic acid (PO₂-OH), derivatives of phosphatidic acid and bioisosters to phosphatic acid and derivatives thereof (among others phosphatidic acid derivatives to which a hydrophilic polymer or polysaccharide is covalently attached).

As mentioned above, preferred embodiments imply that Y is -OC(O)- where Y is connected to R² via the carboxyl atom. The most preferred embodiments imply that X and Z are O and that Y is -OC(O)- where Y is connected to R² via the carboxyl atom. This means that the lipid derivative is a 1-monoether-2-monoester-phospholipid type compound.

Another preferred group of lipid derivatives is the one where the group X is S.

In one embodiment, R¹ and R² are aliphatic groups of the formula Y¹ Y² where Y² is CH₃ or CO₂H, but preferably CH₃, and where Y¹ is -(CH₂)ₙ₁(CH=CH)ₙ₂(CH₂)ₙ₃(CH=CH)ₙ₄-(CH₂)ₙ₅(CH=CH)ₙ₆(CH₂)ₙ₇(CH=CH)ₙ₈(CH₂)ₙ₉; the sum of n1+2n2+n3+2n4+n5+2n6+n7+ 2n8+n9 is an integer of from 9 to 23; that is, the aliphatic group, Y¹Y², is from 10-24 carbon atoms in length. n1 is equal to zero or is an integer of from 1 to 23; n3 is equal to zero or is an integer of from 1 to 20; n5 is equal to zero or is an integer of from 1 to 17; n7 is equal to zero or is an integer of from 1 to 14; n9 is equal to zero or is an integer of from 1 to 11; and each of n2, n4, n6 and 8 is independently equal to zero or 1.

Although the aliphatic groups may be unsaturated and even substituted with halogens (flouro, chloro, bromo, iodo) and C₁₋₄-groups (i.e. yielding branched aliphatic groups), the aliphatic groups as R¹ and R² are in one embodiment preferably saturated as well as unbranched, that is, they preferably have no double bonds between adjacent carbon atoms, each of n2, n4, n6 and n8 then being equal to zero. Accordingly, Y¹ is preferably (CH₂)ₙ₁. More preferably (in this embodiment), R¹ and R² are each independently (CH₂)ₙ₁CH₃, and most preferably, (CH₂)₁₇CH₃ or (CH₂)₁₅CH₃. In alternative embodiments, the groups can have one or more double bonds, that is, they can be unsaturated, and one or more of n2, n4, n6 and n8 can be equal to 1. For example, when the unsaturated hydrocarbon has one double bond, n2 is equal to 1, n4, n6 and n8 are each equal to zero and Y¹ is (CH₂)ₙ₁ CH=CH(CH₂)ₙ₃. n1 is equal to zero or is an integer of from 1 to 21, and n3 is also zero or is an integer of from 1 to 20, at least one of n1 or n3 not being equal to zero.

In one particular embodiment, the lipid derivatives are those which are monoether lipids where X and Z are O, R¹ and R² are independently selected from alkyl groups, (CH₂)ₙCH₃, where n is 11, 12, 13, 14, 15, 16, 17, 18, 19, 20, 21, 22, 23, 24, 25, 26, 27, 28, or 29, preferably 14, 15 or 16, in particular 14; Y is -OC(O)-, Y then being connected to R² via the carbonyl carbon atom.

With respect to the hydrophilic moiety (often known as the "head group") which corresponds to R³, it is believed that a wide variety of groups corresponding to phosphatidic acid (PO₂-OH), derivatives of phosphatidic acid and bioisosters to phosphatic acid and derivatives thereof can be used. As will be evident, the crucial requirement to R³ is that the groups should allow for enzymatic cleavage of the R² group (actually R²-C(=O) or R²-OH) by extracellular PLA₂. "Bioisosters to phosphatidic acid and derivatives thereof" indeed implies that such groups - as phosphatidic acid - should allow for enzymatic cleavage by extracellular PLA₂.

R³ is typically selected from phosphatidic acid (PO₂-OH), phosphatidylcholine (PO₂-O-CH₂CH₂N(CH₃)₃), phosphatidylethanolamine (PO₂-O-CH₂CH₂NH₂), N-methylphosphatidylethanolamine (PO₂-O-CH₂CH₂NCH₂), phosphatidylserine, phosphatidylinositol, and phosphatidylglycerol (PO₂-O-CH₂CHOHCH₂OH). Other possible derivatives of phosphatidic acid are those where dicarboxylic acids, such as glutaric, sebacic, succinic and tartaric acids, are coupled to the terminal nitrogen of phosphatidylethanolamines, phosphatidylserine, phosphatidylinositol, etc.

In the particular embodiment where a fraction of the lipid derivative also is a lipopolymer or glycolipid, a hydrophilic polymer or polysaccharide is typically covalently attached to the phosphatidyl part of the lipid derivative.

Hydrophilic polymers which suitable can be incorporated in the lipid derivatives of the invention so as to form lipopolymers are those which are readily water-soluble, can be covalently attached to a vesicle-forming lipid, and which are tolerated *in vivo* without toxic effects (i.e. are biocompatible). Suitable polymers include polyethylene glycol (PEG), polylactic (also termed polylactide), polyglycolic acid (also termed polyglycolide), a polylactic-polyglycolic acid copolymer, polyvinyl alcohol, polyvinylpyrrolidone, polymethoxazoline, polyethyloxazoline, polyhydroxypropyl methacrylamide, polymethacrylamide, polydimethylacrylamide, and derivatised celluloses such as hydroxymethylcellulose or hydroxyethylcellulose.

Preferred polymers are those having a molecular weight of from about 100 daltons up to about 10,000 daltons, and more preferably from about 300 daltons to about 5,000 daltons. In a particularly preferred embodiment, the polymer is polyethyleneglycol having a molecular weight of from about 100 to about 5,000 daltons, and more preferably having a molecular weight of from about 300 to about 5,000 daltons. In a particularly preferred embodiment, the polymer is polyethyleneglycol of 750 daltons (PEG(750)). Polymers may also be defined by the number of monomers therein; a preferred embodiment of the present invention utilises polymers of at least about three monomers, such PEG polymers consisting of three monomers (approximately 150 daltons).

When the glycolipid or lipopolymer is represented by a fraction of the lipid derivative, such a lipid derivative (lipid derivative with a polymer or polysaccharide chain) typically constitutes 1-80 mol%, such as 2-50 mol% or 3-25 mol% of the total dehydrated lipid-based system. For micellular compositions, however, the fraction may be even higher, such as from 1-100 mol%, such as 10-100 mol%, of the total dehydrated lipid-based system.

Preferred polymers to be covalently linked to the phosphatidyl part (e.g. via the terminal nitrogen of phosphatidylethanolamine) are polyethylene glycol (PEG), polyactide, polyglycolic acid, polyactide-polyglycolic acid copolymer, and polyvinyl alcohol.

One highly interesting aspect of the present invention is the possibility of modifying the pharmaceutical effect of the lipid derivative by modifying the group R². It should be understood that R² should be an organic radical having at least 7 carbon atoms) (such as an aliphatic group having a certain length (at least 7, preferably 9, carbon atoms)), a high degree of variability is possible, e.g. R² need not necessarily to be a long chain residue, but may represent more complex structures.

Generally, it is believed that R² may either be rather inert for the environment in which it can be liberated by extracellular PLA₂ or that R² may play an active pharmaceutical role, typically as an auxiliary drug substance or as an efficiency modifier for the lysolipid derivative and/or any other (second) drug substances present in the environment.

In some embodiments, the group R² will be a long chain residue, e.g. a fatty acid residue (the fatty acid will include a carbonyl from the group Y). This has been described in detail above. Interesting examples of auxiliary drug substances as R² within this subgroups are polyunsaturated acids, e.g. oleate, linoleic, linonleic, as well as derivatives of arachidonoyl (including the carbonyl from Y), e.g. prostaglandins such as prostaglandin E₁, as arachidonic acid derivatives are know regulators of hormone action including the action of prostaglandins, thromboxanes, and leukotrines. Examples of efficiency modifiers as R² are those which enhance the permeability of the target cell membrane as well as enhances the activity of extracellular PLA₂ or the active drug substance or any second drug substances. Examples hereof are short chain (C₈₋₁₂) fatty acids.

However, it is also envisaged that other groups might be useful as the organic radical R², e.g. vitamin D derivatives, steroid derivatives, retinoic acid (including all-trans-retinoic acid, all-cis-retinoic acid, 9-cis-retinoic acid, 13-cis-retinoic acid), cholecalciferol and tocopherol analogues, pharmacologically active carboxylic acids such as branched-chain aliphatic carboxylic acids (e.g. valproic acid and those described in WO 99/02485), salicylic acids (e.g. acetylsalicylic acid), steroidal carboxylic acids (e.g. lysergic and isolysergic acids), monoheterocyclic carboxylic acids (e.g. nicotinic acid) and polyheterocyclic carboxylic acids (e.g. penicillins and cephalosporins), diclofenac, indomethacin, ibuprofen, naproxen, 6-methoxy-2-naphthylacetic acid.

It should be understood that the various examples of possible R² groups are referred to by the name of a discrete species, rather than the name of the radical. Furthermore, it should be understood that the possible examples may include the carbonyl group or oxy group of the bond via which the organic radical is linked to the lipid skeleton (corresponding to "Y" in the formula above). This will of course be appreciated by the person skilled in the art.

Even though it has not specifically been indicated in the general formula for the suitable examples of lipid derivatives to be used within the present invention, it should be understood that the glycol moiety of the lipid derivatives may be substituted, e.g. in order to modify the cleavage rate by extracellular PLA₂ or simply in order to modify the properties of the liposomes comprising the lipid derivatives.

Some of the above defined lipid derivatives may already be known, but is believed that some subgroups thereof are uniquely novel compounds.

A particular group of novel compounds is lipid derivatives of the following formula: 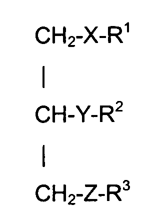 wherein
X and Z independently are selected from O, CH₂, NH, NMe, S, S(O), and S(O)₂, preferably from O, NH, NMe and CH₂, in particular O;
Y is -OC(O)-, Y then being connected to R² via either the oxygen or carbonyl carbon atom, preferably via the carbonyl carbon atom;
R¹ is an aliphatic group of the formula Y¹Y²;
R² is an organic radical having at least 7 carbon atoms;
where Y¹ is -(CH₂)ₙ₁-(CH=CH)ₙ₂-(CH₂)ₙ₃-(CH=CH)ₙ₄-(CH₂)ₙ₅-(CH=CH)ₙ₆-(CH₂)ₙ₇-(CH=CH)ₙ₈ᵣ-(CH₂)ₙ₉, and the sum of n1+2n2+n3+2n4+n5+2n6+n7+2n8+n9 is an integer of from 9 to 29; n1 is zero or an integer of from 1 to 29, n3 is zero or an integer of from 1 to 20, n5 is zero or an integer of from 1 to 17, n7 is zero or an integer of from 1 to 14, and n9 is zero or an integer of from 1 to 11; and each of n2, n4, n6 and n8 is independently zero or 1; and Y² is CH₃ or CO₂H; where each Y¹-Y² independently may be substituted with halogen or C₁₋₄-alkyl, but preferably Y¹-Y² is unsubstituted,
R³ is selected from derivatives of phosphatidic acid to which a hydrophilic polymer or polysaccharide is attached. The hydrophilic polymer or polysaccharide is typically and preferably selected from polyethylene glycol, poly(lactic acid), poly(glycolic acid), poly(lactic acid)-poly(glycolic acid) copolymers, polyvinyl alcohol, polyvinylpyrrolidone, polymethoxazoline, polyethyloxazoline, polyhydroxypropyl methacrylamide, polymethacrylamide, polydimethylacrylamide, and derivatised celluloses, in particular from polyethylene glycol, poly(lactic acid), poly(glycolic acid), poly(lactic acid)-poly(glycolic acid) copolymers, and polyvinyl alcohol.

A particular subgroups are those wherein X and Z are O, R¹ and R² are independently selected from alkyl groups, (CH₂)ₙCH₃, where n is 11, 12, 13, 14, 15, 16, 17, 18, 19, 20, 21, 22, 23, 24, 25, 26, 27, 28, or 29, preferably 14 or 16; Y is -OC(O)-, Y then being connected to R² via the carbonyl carbon atom.

A specific group of compounds are polyethyleneoxide-1-O-palmityl-*sn*-2-palmitoylphosphatidyl ethanolamine, DPPE-PEG, and polyethyleneoxide-1-O-stearyl-*sn*-2-stearoylphosphatidylethanolamine, DSPE-PEG, with PEG molecular weight from 100 to 10000 Daltons, in particular from 300-5000 Daltons.

### fraction

Furthermore, the present invention provides a pharmaceutical composition comprising a pharmaceutically acceptable carrier and a lipid derivative as defined above. Preferably, the lipid derivative in such a composition is dispersed in the form of a liposome (see below).

Also, the present invention relates to such lipid derivatives for use as a medicament, preferably present in a pharmaceutical composition, and to the use of a lipid derivative as defined above for the preparation of a medicament for the treatment of diseases or conditions associated with a localised increase in extracellular phospholipase A2 activity in mammalian tissue. Such diseases or conditions are typically selected from cancer, e.g. a brain, breast, lung, colon or ovarian cancer, or a leukemia, lymphoma, sarcoma, carcinoma, and inflammatory conditions. The present compositions and uses are especially applicable in the instances where the increase in extracellular PLA₂ activity is at least 25% compared to the normal level of activity in the tissue in question, the tissue being that of a mammal, in particular a human.

### Lipid derivatives as prodrugs

As described above, the present invention provides a lipid-based drug delivery system for administration of an active drug substance selected from lysolipid derivatives, wherein the active drug substance is present in the lipid-based system in the form of a prodrug, said prodrug being a lipid derivative having (a) an aliphatic group of a length of at least 7 carbon atoms and an organic radical having at least 7 carbon atoms, and (b) a hydrophilic moiety, said prodrug furthermore being a substrate for extracellular phospholipase A2 to the extent that the organic radical can be hydrolytically cleaved off, whereas the aliphatic group remains substantially unaffected, whereby the active drug substance is liberated in the form of a lysolipid derivative which is not a substrate for lysophospholipase, said system having included therein lipopolymers or glycolipids so as to present hydrophilic chains on the surface of the system.

By the term "active drug substance" is meant any chemical entity which will provide a prophylactic or therapeutic effect in the body of a mammal, in particular a human. Thus, the present invention mainly relates to the therapeutic field.

The term "prodrug" should be understood in the normal sense, namely as a drug which is masked or protected with the purpose of being converted (typically by cleavage, but also by in vivo chemical conversion) to the intended drug substance. The person skilled in the art will recognise the scope of the term "prodrug".

The active drug substance is selected from lysolipid derivatives, and as it will be understood from the present description with claims, the lysolipid derivatives relevant within the present invention will have a therapeutic effect - at least - in connection with diseases and conditions where a local area of the body of the mammal has a level of extracellular PLA₂ activity which can liberate the lysolipid derivative.

As will be understood from the present description with claims, the lipid derivative will often constitute the prodrug referred to above and the lysolipid derivative will thereby constitute the active drug substance often a monoether lysolipid derivative. It should however be understood that this does not exclude the possibility of including other drug substances, referred to as second drug substances, in the drug delivery systems of the invention, neither does it exclude that the organic radical which can be hydrolytically cleaved by the action of extracellular PLA₂ can have a certain pharmaceutical effect (e.g. as an auxiliary drug substance or an efficiency modifier as described elsewhere herein). Furthermore, the pharmaceutical effect of the "active drug substance", i.e. the lysolipid derivative, need not the be the most predominant when a second drug substance is included, actually the effect of the second drug substance might very well be the most predominant as will become apparent in the other main embodiment (see "Lipid derivative liposomes as drug delivery systems", below).

The active drug substance (lipolipid derivative) release from the prodrug (lipid derivative) is believed to take place as illustrated in the following example:

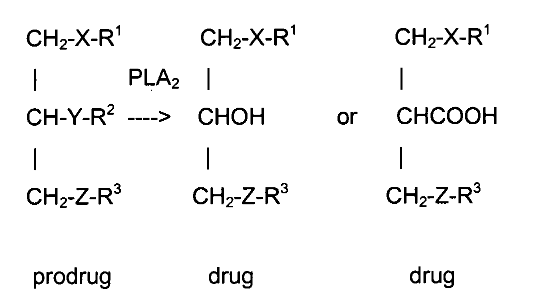

Furthermore, the substituent R² may constitute an auxiliary drug substance or an efficiency modifier for the active drug substance and will simultaneously be released under the action of extracellular PLA₂:

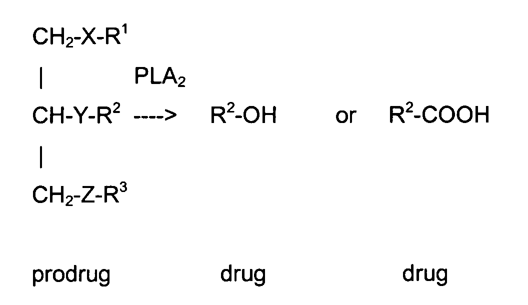

It has been described above under the definition of R² how the group R² can have various independent or synergistic effects in association with the active drug substance, e.g. as an auxiliary drug substance or an efficiency modifier, e.g. permeability or cell lysis modifier. It should be borne in mind that the groups corresponding to R² (e.g. R²-OH or R²-COOH) might have a pharmaceutical effect which is predominant in relation the effect of the lysolipid derivative (active drug substance).

### Lipid derivatives formulated as liposomes and micelles

The term "lipid-based drug delivery system" should encompass macromolecular structures which as the main constituent include lipid or lipid derivatives. Suitable examples hereof are liposomes and micelles. It is presently believed that liposomes offer the broadest scope of applications and those have been described most detailed in the following. Although liposomes currently are believed to be the preferred lipid-based system, micellular systems are also believed to offer interesting embodiments within the present invention.

In one important variant which advantageously can be combined with the embodiments described herein, the lipid derivative (e.g. the prodrug) is included in liposomes either as the only constituent or - which is more common - in combination with other constituents (other lipids, sterols, etc.). Thus, the lipid-based systems described herein are preferably in the form of liposomes, wherein the liposomes are build up of layers comprising the lipid derivative (e.g. a prodrug).

"Liposomes" are known as self-assembling structures comprising one or more lipid bilayers, each of which surrounds an aqueous compartment and comprises two opposing monolayers of amphipathic lipid molecules. Amphipathic lipids (herein i.a. lipid derivatives) comprise a polar (hydrophilic) headgroup region (corresponding to the substituent R³ in the lipid derivatives) covalently linked to one or two non-polar (hydrophobic) aliphatic groups (corresponding to R¹ and R² in the lipid derivatives). Energetically unfavourable contacts between the hydrophobic groups and the aqueous medium are generally believed to induce lipid molecules to rearrange such that the polar headgroups are oriented towards the aqueous medium while the hydrophobic groups reorient towards the interior of the bilayer. An energetically stable structure is formed in which the hydrophobic groups are effectively shielded from coming into contact with the aqueous medium.

Liposomes can have a single lipid bilayer (unilamellar liposomes, "ULVs"), or multiple lipid bilayers (multilamellar liposomes, "MLVs"), and can be made by a variety of methods (for a review, see, for example, Deamer and Uster, Liposomes, Marcel Dekker, N.Y., 1983, 27-52). These methods include Bangham's methods for making multilamellar liposomes (MLVs); Lenk's, Fountain's and Cullis' methods for making MLVs with substantially equal interlamellar solute distribution (see, e.g., US 4,522,803, US 4,588,578, US 5,030,453, US 5,169,637 and US 4,975,282); and Papahadjopoulos et al.'s reverse-phase evaporation method (US 4,235,871) for preparing oligolamellar liposomes. ULVs can be produced from MLVs by such methods as sonication (see Papahadjopoulos et al., Biochem. Biophys. Acta, 135, 624 (1968)) or extrusion (US 5,008,050 and US 5,059,421).

The liposome of this invention can be produced by the methods of any of these disclosures, the contents of which are incorporated herein by reference.

Various methodologies, such as sonication, homogenisation, French Press application and milling can be used to prepare liposomes of a smaller size from larger liposomes. Extrusion (see US 5,008,050) can be used to size reduce liposomes, that is to produce liposomes having a predetermined mean size by forcing the liposomes, under pressure, through filter pores of a defined, selected size. Tangential flow filtration (see WO 89/08846), can also be used to regularise the size of liposomes, that is, to produce liposomes having a population of liposomes having less size heterogeneity, and a more homogeneous, defined size distribution. The contents of these documents are incorporated herein by reference. Liposome sizes can also be determined by a number of techniques, such as quasi-electric light scattering, and with equipment, e.g., Nicomp® particle sizers, well within the possession of ordinarily skilled artisans.

It is quite interesting to note that the lipid derivatives of the present invention can constitute the major part of a lipid-based system even if this system is a liposome system. This fact resides in the structural (but not functional) similarity between the lipid derivatives of the present invention and lipids. Thus, it is believed that the lipid derivatives for the present invention can be the sole constituent of liposomes, i.e. up to 100 mol% of the total dehydrated liposomes can be constituted by the lipid derivatives. This is in contrast to the known mono-ether lysolipides, which can only constitute a minor part of the liposomes.

Typically, as will be described in detail below, liposomes advantageously include other constituents which may or may not have a pharmaceutical effect, but which will render the liposome structure more stable (or alternatively more unstable) or will protect the liposomes against clearance and will thereby increase the circulation time thereby improving the overall efficiency of a pharmaceutical including the liposome.

This being said, it is believed that the particular lipid derivatives will typically constitute from 15-100 mol%, such as 50-100 mol%, preferably from 75-100 mol%, in particular 90-100 mol%, based on the total dehydrated liposome.

The liposomes can be unilamellar or multilamellar. Some preferred liposomes are unilamellar and have diameters of less than about 200 nm, more preferably, from greater than about 50 nm to less than about 200 nm.

The liposomes are typically - as known in the art - prepared by a method comprising the steps of: (a) dissolving the lipid derivative in an organic solvent; (b) removing the organic solvent from the lipid derivative solution of step (a); and (c) hydrating the product of step (b) with an aqueous solvent so as to form liposomes.

The method may further comprise a step of adding an second drug substance (see below) to the organic solvent of step (a) or the aqueous phase of step (c).

Subsequently, the method may comprise a step of extruding the liposomes produced in step (c) through a filter to produce liposomes of a certain size, e.g. 100 nm.

Lipid-based particulate systems, i.e. liposomes as well as micelles; of sizes covering a broad range may be prepared according to the above-mentioned techniques. Depending on the route of administration, suitable sizes for pharmaceutical applications will normally be in the range of 20-10,000 nm, in particular in the range of 30-1000 nm. Sizes in the range of 50-200 nm are normally preferred because liposomes in this size range are generally believed to circulate longer in the vascular system of mammals than do larger liposomes which are more quickly recognised by the mammals' reticuloendothelial systems ("RES"), and hence, more quickly cleared from the circulation. Longer vascular circulation can enhance therapeutic efficacy by allowing more liposomes to reach their intended site of actions, e.g., tumours or inflammations.

It is believed that for a drug delivery system as defined in the embodiments herein, which is adapted to be administered via intraveneous and intramuscular injection, the liposomes should preferably have a mean particle size of about 100 nm. Thus, the particle size should generally be in the range of 50-200 nm.

Furthermore, for a drug delivery system adapted to be administered via subcutaneous injection, the liposomes should preferably have a mean particle size from 100 to 5000 nm, and the liposomes can then be uni- or multilayered.

One of the advantages by including the lipid derivatives in liposomes is that the liposome structure, in particular when stabilised as described in the following, will have a much longer vascular circulation time that the lipid derivatives as discrete compounds. Furthermore, the lipid derivatives will become more or less inert or even "invisible" when "packed" in liposomes in which lipopolymers or glycolipids are included. This means than any potential disadvantageous effect, e.g. hemolytic effect, can be suppressed.

The liposomes should preferably act as inert constituents until they react the area of interest, e.g. cancerous, infected or inflammatorily diseased areas or tissue. As will be described in the following, liposomes may include a number of other constituents. In particular, a drug delivery system according to the invention may further contain a component which controls the release of any second drug substance, extracellular PLA₂ activity controlling agents or permeability enhancer, e.g. short chain lipids and lipopolymers/glycolipids.

Two very important groups of compounds to be included in liposomes as modifiers are the stabilising compound lipopolymers and glycolipids, such as lipopolymers (e.g. polyethyleneoxide-dipalmitoylphosphatidyl ethanolamine, DPPE-PEG, polyethyleneoxidedistearoylphosphatidylethanolamine, DSPE-PEG) with PEG molecular weight from 100 to 10000 Daltons. It has been shown that lipopolymers function as stabilisers for the liposome, i.e. lipopolymer increases the circulation time, and - which is highly interesting in the present context, as activators for extracellular PLA₂. The stabilising effect will be described in the following.

Liposome outer surfaces are believed to become coated with serum proteins, such as opsonins, in mammals' circulatory systems. Without intending in any way to be limited by any particular theory, it is believed that liposome clearance can be inhibited by modifying the outer surface of liposomes such that binding of serum proteins thereto is generally inhibited. Effective surface modification, that is, alterations to the outer surfaces of liposomes which result in inhibition of opsonisation and RES uptake, is believed to be accomplished by incorporating into liposomal bilayers lipids whose polar headgroups have been derivatised by attachment thereto of a chemical moiety which can inhibit the binding of serum proteins to liposomes such that the pharmacokinetic behaviour of the liposomes in the circulatory systems of mammals is altered and the activity of extracellular PLA₂ is enhanced as described for the lipopolymers above.

Liposome preparations have been devised which avoid rapid RES uptake and which thus have an increased half-life in the bloodstream. STEALTH® liposomes (Liposome Technology Inc., Menlo Park, Calif.) include polyethyleneglycol (PEG)-grafted lipids at about 5 mol% of the total dehydrated liposome . The presence of polymers on the exterior liposome surface decreases the uptake of liposomes by the organs of the RES. The liposome membranes can be constructed so as to resist the disruptive effects of the surfactant contained therein. For example, a liposome membrane which contains as constituents lipids derivatised with a hydrophilic (i.e., water-soluble) polymer normally has increased stability. The polymer component of the lipid bilayer protects the liposome from uptake by the RES, and thus the circulation time of the liposomes in the bloodstream is extended.

Hydrophilic polymers suitable for use in lipopolymers are those which are readily water-soluble, can be covalently attached to a vesicle-forming lipid, and which are tolerated in vivo without toxic effects (i.e., are biocompatible). Suitable polymers include polyethylene glycol (PEG), polylactic (also termed polylactide), polyglycolic acid (also termed polyglycolide), a polylactic-polyglycolic acid copolymer, and polyvinyl alcohol. Preferred polymers are those having a molecular weight of from about 100 or 120 daltons up to about 5,000 or 10,000 daltons, and more preferably from about 300 daltons to about 5,000 daltons. In a particularly preferred embodiment, the polymer is polyethyleneglycol having a molecular weight of from about 100 to about 5,000 daltons, and more preferably having a molecular weight of from about 300 to about 5,000 daltons. In a particularly preferred embodiment, the polymer is polyethyleneglycol of 750 daltons (PEG(750)). Polymers may also be defined by the number of monomers therein; a preferred embodiment of the present invention utilises polymers of at least about three monomers, such PEG polymers consisting of three monomers (approximately 150 daltons). Other hydrophilic polymers which may be suitable for use in the present invention include polyvinylpyrrolidone, polymethoxazoline, polyethyloxazoline, polyhydroxypropyl methacrylamide, polymethacrylamide, polydimethylacrylamide, and derivatised celluloses such as hydroxymethylcellulose or hydroxyethylcellulose.

Glycolipids are lipids to which a hydrophilic polysaccharide chain is covalently attached. It will be appreciated that glycolipids can be utilised like lipopolymers although the lipopolymers currently presents the most promising results.

It is generally believed that the content of lipopolymer advantageously will be in the range of 1-50 mol%, such as 2-25%, in particular 2-15 mol%, based on the total dehydrated liposome.
The liposomes' bi- or multilayers may also contain other constituents such as other lipids, sterolic compounds, polymer-ceramides as stabilisers and targeting compounds, etc.

The liposomes comprising lipid derivatives may (in principle) exclusively consist of the lipid derivatives. However, in order to modify the liposomes, "other lipids" may be included as well. Other lipids are selected for their ability to adapt compatible packing conformations with the lipid derivative components of the bilayer such that the all the lipid constituents are tightly packed, and release of the lipid derivatives from the bilayer is inhibited. Lipid-based factors contributing to compatible packing conformations are well known to ordinarily skilled artisans and include, without limitation, acyl chain length and degree of unsaturation, as well as the headgroup size and charge. Accordingly, suitable other lipids, including various phosphatidylethanolamines ("PE's") such as egg phosphatidylethanolamine ("EPE") or dioleoyl phosphatidylethanolamine ("DOPE"), can be selected by ordinarily skilled artisans without undue experimentation. Lipids may be modified in various way, e.g. by headgroup derivatisation with dicarboxylic acids, such as glutaric, sebacic, succinic and tartaric acids, preferably the dicarboxylic acid is glutaric acid ("GA"). Accordingly, suitable headgroup-derivatised lipids include phosphatidylethanolamine-dicarboxylic acids such as dipalmitoyl phosphatidylethanolamine-glutaric acid ("DPPE-GA"), palmitoyloleoyl phosphatidylethanolamine-glutaric acid ("POPE-GA") and dioleoyl phosphatidylethanolamine-glutaric acid ("DOPE-GA"). Most preferably, the derivatised lipid is DOPE-GA.

The total content of "other lipids" will typically be in the range of 0-30 mol%, in particular 1-10 mol%, based on the total dehydrated liposome.

Sterolic compound included in the liposome may generally affects the fluidity of lipid bilayers. Accordingly, sterol interactions with surrounding hydrocarbon groups generally inhibit emigration of these groups from the bilayer. An examples of a sterolic compound (sterol) to be included in the liposome is cholesterol, but a variety of other sterolic compounds are possible. It is generally believed that the content of sterolic compound, if present, will be in the range of 0-25 mol%, in particular 0-10 mol%, such as 0-5 mol%, based on the total dehydrated liposome.

Polymer-ceramides are stabilisers improving the vascular circulation time. Examples are polyethylene glycol derivatives of ceramides (PEG-ceramides), in particular those where the molecular weight of the polyethylene glycol is from 100 to 5000. It is generally believed that the content of polymer-ceramides, will be in the range of 0-30 mol%, in particular 0-10 mol%, based on the total dehydrated liposome.

Still other ingredients may constitute 0-2 mol%, in particular 0-1 mol%, based on the total dehydrated liposome.

According to an embodiment of the present invention, the lipid bilayer of a liposome contains lipids derivatised with polyethylene glycol (PEG), such that the PEG chains extend from the inner surface of the lipid bilayer into the interior space encapsulated by the liposome, and extend from the exterior of the lipid bilayer into the surrounding environment (see e.g. US 5,882,679 and Figs. 10 and 11).

A variety of coupling methods for preparing a vesicle-forming lipid derivatised with a biocompatible, hydrophilic polymer such as polyethylene glycol are known in the art (see, e.g., US 5,213,804; US 5,013,556).

The derivatised lipid components of liposomes according to the present invention may additionally include a labile lipid-polymer linkage, such as a peptide, ester, or disulfide linkage, which can be cleaved under selective physiological conditions, such as in the presence of peptidase or esterase enzymes or reducing agents. Use of such linkages to couple polymers to phospholipids allows the attainment of high blood levels of such liposomes for several hours after administration, followed by cleavage of the reversible linkages and removal of the polymer from the exterior liposome bilayer. The polymer-less liposomes are then subject to rapid uptake by the RES system (see, e.g., US 5,356,633).

Additionally, liposomes according to the present invention may contain non-polymer molecules bound to the exterior of the liposome, such as haptens, enzymes, antibodies or antibody fragments, cytokines and hormones (see, e.g., US 5,527,528), and other small proteins, polypeptides, single sugar polysaccharide moieties, or non-protein molecules which confer a particular enzymatic or surface recognition feature to the liposome. See published PCT application WO 94/21235. Surface molecules which preferentially target the liposome to specific organs or cell types are referred to herein as "targeting molecules" and include, for example, antibodies and sugar moieties, e.g. gangliosides or those based on mannose and galactose, which target the liposome to specific cells bearing specific antigens (receptors for sugar moieties). Techniques for coupling surface molecules to liposomes are known in the art (see, e.g., US 4,762,915).

The liposome can be dehydrated, stored and then reconstituted such that a substantial portion of its internal contents is retained. Liposomal dehydration generally requires use of a hydrophilic drying protectant such as a disaccharide sugar at both the inside and outside surfaces of the liposome bilayers (see US 4,880,635). This hydrophilic compound is generally believed to prevent the rearrangement of the lipids in the liposome, so that the size and contents are maintained during the drying procedure and through subsequent rehydration. Appropriate qualities for such drying protectants are that they are strong hydrogen bond acceptors, and possess stereochemical features that preserve the intramolecular spacing of the liposome bilayer components. Alternatively, the drying protectant can be omitted if the liposome preparation is not frozen prior to dehydration, and sufficient water remains in the preparation subsequent to dehydration.

### Lipid derivative liposomes as drug carrier systems

As mentioned above, the liposomes including the lipid derivatives of the present invention may also include second drug substances. In a particular embodiment, the lipid-based drug delivery system described above is in the form of liposomes wherein a second drug substance is incorporated. It should be understood that second drug substances may comprise pharmaceutically active ingredients which may have an individual or synergistic pharmaceutical effect in combination with the lipid derivative and lysolipid derivatives. The term "second" does not necessarily imply that the pharmaceutical effect of the second drug substance is inferior in relation to that of, e.g., the active drug substance derived from the prodrug, but is merely used to differentiate between the two groups of substances.

This being said, the present invention also provides a drug delivery system which is in the form of liposomes, and wherein a second drug substance is incorporated.

A possible "second drug substance" is any compound or composition of matter that can be administered to mammals, preferably humans. Such agents can have biological activity in mammals. Second drug substances which may be associated with liposomes include, but are not limited to: antiviral agents such as acyclovir, zidovudine and the interferons; antibacterial agents such as aminoglycosides, cephalosporins and tetracyclines; antifungal agents such as polyene antibiotics, imidazoles and triazoles; antimetabolic agents such as folic acid, and purine and pyrimidine analogs; antineoplastic agents such as the anthracycline antibiotics and plant alkaloids; sterols such as cholesterol; carbohydrates, e.g., sugars and starches; amino acids, peptides, proteins such as cell receptor proteins, immunoglobulins, enzymes, hormones, neurotransmitters and glycoproteins; dyes; radiolabels such as radioisotopes and radioisotope-labeled compounds; radiopaque compounds; fluorescent compounds; mydriatic compounds; bronchodilators; local anesthetics; and the like.

Liposomal second drug substance formulations enhance the therapeutic index of the second drug substances by reducing the toxicity of the drug. Liposomes can also reduce the rate at which a second drug substance is cleared from the vascular circulation of mammals. Accordingly, liposomal formulation of second drug substance can mean that less of the drug need be administered to achieve the desired effect.

Liposomes can be loaded with one or more second drug substances by solubilising the drug in the lipid or aqueous phase used to prepare the liposomes. Alternatively, ionisable second drug substances can be loaded into liposomes by first forming the liposomes, establishing an electrochemical potential, e.g., by way of a pH gradient, across the outermost liposomal bilayer, and then adding the ionisable second drug substance to the aqueous medium external to the liposome (see, e.g., US 5,077,056 and WO 86/01102).

Methods of preparing lipophilic drug derivatives which are suitable for liposome or micelle formulation are known in the art (see e.g., US 5,534,499 and US 6,118,011 describing covalent attachment of therapeutic agents to a fatty acid chain of a phospholipid). A micellar formulation of taxol is described in Alkan-Onkyuksel et al., Pharmaceutical Research, 11:206 (1994).

Accordingly, the second drug substance may be any of a wide variety of known and possible pharmaceutically active ingredients, but is preferably a therapeutically and/or prophylactically active substance. Due to the mechanism involved in the degradation of the liposomes of the present invention, it is preferred that the second drug substance is one relating to diseases and/or conditions associated with a localised increase in extracellular PLA₂ activity.

Particularly interesting second drug substances are selected from (i) antitumour agents such as anthracyline derivatives, cisplatin, paclitaxel, 5-fluoruracil, exisulind, cis-retinoic acid, suldinac sulfide and vincristine, (ii) antibiotics and antifungals, and (iii) antiinflammatory agents such as steroids and non-steroids. In particular the steroids can also have a stabilising effect on the liposomes.

The cytotoxic effects of a broad range of anticancer agents are likely to improve when encapsulated in the carriers of this invention. Furthermore, it is expected that the hydrolysis products, i.e. monoether lysolipids and ester-linked derivatives, act in turn as absorption enhancers for drug permeation across the target membranes when the carriers locally are broken down in the diseased tissue.

It is envisaged that the second drug substance will be distributed in the liposomes according to their hydrophilicity, i.e. hydrophilic second drug substances will tend to be present in the cavity of the liposomes and hydrophobic second drug substances will tend to be present in the hydrophobic bilayer. Method for incorporation of second drug substances are know in the art as has been made clear above.

It should be understood from the above, that the lipid derivatives may - as prodrugs or discrete constituents - posses a pharmaceutical activity. However, in a particular embodiment, the present invention furthermore relates to a lipid based drug delivery system for administration of an second drug substance, wherein the second drug substance is incorporated in the system (e.g. where the second drug substance is encapsulated in the interior of the liposome or in the membrane part of the liposome or the core region of micelle), said system including lipid derivatives which has (a) an aliphatic group of a length of at least 7 carbon atoms and an organic radical having at least 7 carbon atoms, and (b) a hydrophilic moiety, where the lipid derivative furthermore is a substrate for extracellular phospholipase A2 to the extent that the organic radical can be hydrolytically cleaved off, whereas the aliphatic group remains substantially unaffected, so as to result in an organic acid fragment or an organic alcohol fragment and a lysolipid fragment, said lysolipid fragment not being a substrate for lysophospholipase, said system having included therein lipopolymers or glycolipids so as to present hydrophilic chains on the surface of the system.

As above for the system according to the other embodiment, the organic radical which can be hydrolytically cleaved off, may be an auxiliary drug substance or an efficiency modifier for the second drug substance. It should be understood that the lipid derivative is a lipid derivative as defined further above. Typically, the lipid derivative constitutes 15-100 mol%, such as 50-100 mol%, of the total dehydrated (liposome) system.

As should be understood from the above, the present invention also provides a pharmaceutical composition comprising a pharmaceutically acceptable carrier and any of the lipid-based drug delivery systems described above. The composition will be described in detail below.

The present invention also relates to the use of any of the lipid-based drug delivery systems described herein as a medicament, and to the use of any of the lipid-based drug delivery systems described herein for the preparation of a medicament for the treatment of diseases or conditions associated with a localised increase in extracellular phospholipase A2 activity in mammalian tissue. Such diseases or conditions are typically selected from cancer, e.g. a brain, breast, lung, colon or ovarian cancer, or a leukemia, lymphoma, sarcoma, carcinoma and inflammatory conditions . Also included is the prophylactic use. The present compositions and uses are especially applicable in the instances the increase in extracellular PLA₂ activity is at least 25% compared to the normal level of activity in the tissue in question, the tissue being that of a mammal, in particular a human.

### Pharmaceutical preparations and therapeutic uses

Also provided herewith is a pharmaceutical composition comprising a pharmaceutically acceptable carrier and the lipid derivative, e.g. as a liposome, of this invention. "Pharmaceutically acceptable carriers" as used herein are those media generally acceptable for use in connection with the administration of lipids and liposomes, including liposomal drug formulations, to mammals, including humans. Pharmaceutically acceptable carriers are generally formulated according to a number of factors well within the purview of the ordinarily skilled artisan to determine and account for, including without limitation: the particular active drug substance and/or second drug substance used, the liposome preparation, its concentration, stability and intended bioavailability; the disease, disorder or condition being treated with the liposomal composition; the subject, its age, size and general condition; and the composition's intended route of administration, e.g., nasal, oral, ophthalmic, subcutaneous, intramammary, intraperitoneal, intravenous, or intramuscular. Typical pharmaceutically acceptable carriers used in parenteral drug administration include, for example, D5W, an aqueous solution containing 5% weight by volume of dextrose, and physiological saline. Pharmaceutically acceptable carriers can contain additional ingredients, for example those which enhance the stability of the active ingredients included, such as preservatives and anti-oxidants.

The liposome or lipid derivative is typically formulated in a dispersion medium, e.g. a pharmaceutically acceptable aqueous medium.

An amount of the composition comprising an anticancer effective amount of the lipid derivative, typically from about 0.1 to about 1000 mg of the lipid derivative per kg of the mammal's body, is administered, preferably intravenously. For the purposes of this invention, "anticancer effective amounts" of liposomal lipid derivatives are amounts effective to inhibit, ameliorate, lessen or prevent establishment, growth, metastasis or invasion of one or more cancers in mammals to which the lipid derivatives have been administered. Anticancer effective amounts are generally chosen in accordance with a number of factors, e.g., the age, size and general condition of the subject, the cancer being treated and the intended route of administration, and determined by a variety of means, for example, dose ranging trials, well known to, and readily practised by, ordinarily skilled artisans given the teachings of this invention. Antineoplastic effective amounts of the liposomal drugs/prodrugs of this invention are about the same as such amounts of free, nonliposomal, drugs/prodrugs, e.g., from about 0.1 mg of the lipid derivative per kg of body weight of the mammal being treated to about 1000 mg per kg.

Preferably, the liposome administered is a unilamellar liposome having an average diameter of from about 50 nm to about 200 nm. The anti-cancer treatment method can include administration of one or more second drug substances in addition to the liposomal drug, these additional agents being included in the same liposome as the lipid derivative. The second drug substances, which can be entrapped in liposomes' internal compartments or sequestered in their lipid bilayers, are preferably, but not necessarily, anticancer agents.

The pharmaceutical composition is preferably administered parenterally by injection, infusion or implantation (intravenous, intramuscular, intraarticular, subcutaneous or the like) in dosage forms, formulations or e.g. suitable delivery devices or implants containing conventional, non-toxic pharmaceutically acceptable carriers and adjuvants.

The formulation and preparation of such compositions is well-known to those skilled in the art of pharmaceutical formulation. Specific formulations can be found in the textbook entitled "Remington's Pharmaceutical Sciences".

Thus, the pharmaceutical compositions according to the invention may comprise the active drug substances in the form of a sterile injection. To prepare such a composition, the suitable active drug substances are dispersed in a parenterally acceptable liquid vehicle which conveniently may comprise suspending, solubilising, stabilising, pH-adjusting agents and/or dispersing agents. Among acceptable vehicles that may be employed are water, water adjusted to a suitable pH by addition of an appropriate amount of hydrochloric acid, sodium hydroxide or a suitable buffer, 1,3-butanediol, Ringer's solution and isotonic sodium chloride solution. The aqueous formulation may also contain one or more preservatives, for example, methyl, ethyl or n-propyl p-hydroxybenzoate.

Where treatment of a tumour or neoplasm is desired, effective delivery of a liposome-encapsulated drug via the bloodstream requires that the liposome be able to penetrate the continuous (but "leaky") endothelial layer and underlying basement membrane surrounding the vessels supplying blood to a tumour. Liposomes of smaller sizes have been found to be more effective at extravasation into tumours through the endothelial cell barrier and underlying basement membrane which separates a capillary from tumour cells.

As used herein, "solid tumours" are those growing in an anatomical site other than the bloodstream (in contrast to blood-borne tumours such as leukemias). Solid tumours require the formation of small blood vessels and capillaries to nourish the growing tumour tissue.

In accordance with the present invention, the anti-tumour or anti-neoplastic agent of choice is entrapped within a liposome according to the present invention; the liposomes are formulated to be of a size known to penetrate the endothelial and basement membrane barriers. The resulting liposomal formulation can be administered parenterally to a subject in need of such treatment, preferably by intravenous administration. Tumours characterised by an acute increase in permeability of the vasculature in the region of tumour growth are particularly suited for treatment by the present methods. The liposomes will eventually degrade due to lipase action at the tumour site, or can be made permeable by, for example, thermal or ultrasonic radiation. The drug is then released in a bioavailable, transportable solubilised form. Furthermore, a small elevation in temperature as often seen in diseased tissue may further increase the stimulation of extracellular PLA₂.

Where site-specific treatment of inflammation is desired, effective liposome delivery of an drug requires that the liposome have a long blood half-life, and be capable of penetrating the continuous endothelial cell layer and underlying basement membrane surrounding blood vessels adjacent to the site of inflammation. Liposomes of smaller sizes have been found to be more effective at extravasation through the endothelial cell barrier and into associated inflamed regions. However, the limited drug-carrying capacity of conventional small liposome preparations has limited their effectiveness for such purposes.

In accordance with the present invention, the anti-inflammatory agent of choice is entrapped within a liposome according to the present invention; the liposomes are formulated to be of a size known to penetrate the endothelial and basement membrane barriers. The resulting liposomal formulation can be administered parenterally to a subject in need of such treatment, preferably by intravenous administration. Inflamed regions characterised by an acute increase in permeability of the vasculature in the region of inflammation are particularly suited for treatment by the present methods.

It is known that the activity of extracellular PLA₂ is abnormally high in areas of the mammalian body diseased by cancer, inflammation, etc. The present invention have provided a way of exploiting this fact, and it is believed that the extracellular PLA₂ activity should be at least 25% higher in the diseases area of the body (determined in the extracellular environment) compared with a comparative normal area. It is however envisaged that the level of extracellular PLA₂ activity often is much higher, e.g. at least 100%, e.g. at least 200% such as at least 400%. This means that treatment of a mammal in need of a treatment with the purpose of cure or relief, can be conducted with only minimal influence on tissue having a "normal" level of extracellular PLA₂ activity. This is extremely relevant in particular with the treatment of cancer where rather harsh drug (second drug substances) are often needed.

Residing in the realisations behind the present invention, the invention thus provides to a method for selectively drug targeting to diseased areas, such as areas comprising neoplastic cells, e.g., areas within the mammalian body, preferably a human, having a extracellular phospholipase A2 (extracellular PLA₂) activity which is at least 25% higher compared to the normal activity in said areas, by administering to the mammal in need thereof an efficient amount of a drug delivery system defined herein.

Provided is also a method of treating of a mammal afflicted with a cancer, e.g., a brain, breast, lung, colon or ovarian cancer, or a leukemia, lymphoma, sarcoma, carcinoma, which comprises administering a pharmaceutical composition of this invention to the mammal. It is believed that the lipid derivatives and/or second drug substance in liposome form is selectively cytotoxic to tumour cells.

### Toxicity

Toxicity of the liposomes comprising the lipid derivatives can be assessed by determining the therapeutic window "TW", which is a numerical value derived from the relationship between the compound's induction of hemolysis and its ability to inhibit the growth of tumour cells. TW values are defined as Hl₅/Gl₅₀ (wherein "Hl₅" equals the concentration of compound inducing the hemolysis of 5% of the red blood cells in a culture, and wherein "Gl₅₀" equals the dose of compound inducing fifty percent growth inhibition in a population of cells exposed to the agent). The higher an agent's Hl₅ value, the less hemolytic is the agent - higher Hl₅'s mean that greater concentrations of compound are required to be present in order for the compound to induce 5% hemolysis. Hence, the higher its Hl₅, the more therapeutically beneficial is a compound, because more of it can be given before inducing the same amount of hemolysis as an agent with a lower Hl₅. By contrast, lower Gl₅₀ 's indicate better therapeutic agents - a lower Gl₅₀ value indicates that a lesser concentration of an agent is required for 50% growth inhibition. Accordingly, the higher is its Hl₅ value and the lower is its Gl₅₀ value, the better are a compound's agent's therapeutic properties.

Generally, when a drug's TW is less than 1, it cannot be used effectively as a therapeutic agent. That is, the agent's Hl₅ value is sufficiently low, and its Gl₅₀ value sufficiently high, that it is generally not possible to administer enough of the agent to achieve a sufficient level of tumour growth inhibition without also attaining an unacceptable level of hemolysis. As the lipid derivative liposomes take advantage of the lower extracellular PLA₂ activity in the bloodstream compared to the activity in the diseased tissue, it is believed that the TW will be much higher that for normal monoether lysolipids. As the variance in activity is in orders of magnitude and as the liposomes will be "trapped" in tissue with a high extracellular PLA₂ activity, it is generally believed the TW of the liposomes of the invention will be greater than about 3, more preferably greater than about 5, and still more preferably greater than about 8.

The invention will be illustrated by the following non-limiting examples.

### EXAMPLES

### Example 1

### Liposome preparation

Unilamellar fully hydrated liposomes with a narrow size distribution were made from 1-O-hexadecyl-2-hexadecanoyl-*sn*-glycero-3-phosphocholine (1-O-DPPC) and di-hexadecanoyl-*sn*-glycero-3-phosphocholine (DPPC). DPPC were obtained from Avanti Polar lipids and 1-O-DPPC were synthesised in our laboratory. Briefly, weighed amounts of DPPC or 1-O-DPPC were dissolved in chloroform. The solvent was removed by a gentle stream of N₂ and the lipid films were dried overnight under low pressure to remove trace amounts of solvent. Multilamellar vesicles were made by dispersing the dried lipids in a buffer solution containing: 150 mM KCL, 10 mM HEPES (pH = 7.5), 1 mM NaN₃, 30 µM CaCl₂ and 10 µM EDTA. The multilamellar vesicles were extruded ten times through two stacked 100 nm pore size polycarbonate filters as described by Mayer et al., *Biochim. Biophys. Acta,* **858,** 161-168.

Heat capacity curves were obtained using a N-DSC II differential scanning calorimetor (Calorimetry Sciences Corp., Provo) of the power compensating type with a cell volume of 0.34 mL. Before scanning, the liposome suspension was equilibrated for 50 min in the calorimeter at the starting temperature. A scan rate of +10 °C/h was used. The lipid concentration was 1 mM. The gel-to-fluid transition of the multilamellar liposomes (MLV) is characterised as a sharp first-order transition, as reflected by the narrow peak in the heat capacity curves shown in Figs. 1a and 1b (upper curves) for 1-O-DPPC and DPPC. The sharp peak reflects the transitional behaviour of multilamellar liposomes and is in contrast to the broader gel-to-fluid transition observed for unilamellar liposomes (LUV) (Pedersen et al., 1996, *Biophys. J.* **71**, 554-560) as shown in Figs. 1a and 1b (lower curves) for the unilamellar extruded 1-O-DPPC and DPPC liposomes.

### Example 2

### Phospholipase A₂ reaction profile and lag time measurements

Purified snake-venom phospholipase A₂ (PLA₂ from *Agikistrodon piscivorus piscivorus)* has been isolated according to the procedure of Maraganore et al., *J. Biol. Chem.* **259,** 13839-13843. This PLA₂ enzyme belongs to the class of low-molecular weight 14kD secretory enzymes which display structural similarity to human extracellular phospholipase A₂ indicating a common molecular mechanisms of the phospholipase catalysed hydrolysis at the lipid-membrane interface (Wery et al., *Nature* **352**, 79-82; Hønger et al. *Biochemistry* **35**, 9003-9006; Vermehren et al., *Biochimica et Biophysica Acta* **1373**, 27-36). Unilamellar fully hydrated liposomes with a narrow size distribution were prepared from 1-O-hexadecyl-2-hexadecanoyl-*sn*-glycero-3-phosphocholine (1-O-DPPC) and from 1-O-DPPC with 5 mol% 1-O-hexadecyl-2-hexadecanoyl-*sn*-glycero-3-phosphoethanolamine-N-[methoxy(polyethylene glycol)-350] (1-O-DPPE-PEG350) or 5 mol% 1-O-hexadecyl-2-hexadecanoyl-*sn*-glycero-3-phosphoethanolamine-N-[methoxy(polyethylene glycol)-2000] (1-O-DPPE-PEG2000) as described above. Assay conditions for the PLA₂ reaction time profile shown in Fig. 2 and the lag-time and percent hydrolysis reported in Table 1 were: 0.15 mM unilamellar liposomes, 150 nM PLA₂, 150 mM KCL, 10 mM HEPES (pH 7.5), 1 mM NaN₃, 30 µM CaCl₂, and 10 µM EDTA.

| Composition | Lag-time (sec) | 1-O-DPPC (%) |
|---|---|---|
| 100% 1-O-DPPC | 583 | 79 |
| 95% 1-O-DPPC/ 5 % 1-O-DPPE-PEG350 | 128 | 73 |
| 90% 1-O-DPPC/ 10 % 1-O-DPPE-PEG350 | 26 | 75 |
| 95% 1-O-DPPC/ 5 % 1-O-DPPE-PEG2000 | 450 | 56 |
| 90% 1-O-DPPC/ 10 % 1-O-DPPE-PEG2000 | 20 | 89 |

Table 1. Lag-time and percent hydrolysed 1-O-DPPC at 41°C as determined by HPLC. The lipid concentration was 0.150 mM in a 10 mM HEPES-buffer (pH = 7.5).

The catalytic reaction was initiated by adding 8.9 µL of a 42 µM PLA₂ (150 nM) stock solution to 2.5 ml of the thermostated liposome suspension (0.150 mM) equilibrated for 800 sec prior to addition of PLA₂. The characteristic lag-burst behaviour of PLA₂ towards the liposomes is signalled by a sudden increase in the intrinsic fluorescence from PLA₂ at 340 nm after excitation at 285 nm followed by a concomitant decrease in the 90° light scattering from the lipid suspension (Hønger et al., *Biochemistry* **35**, 9003-9006). Samples for HPLC analysis of the amount of non-hydrolysed 1-O-DPPC remaining and consequently the amount of 1-O-hexadecyl-2-hydroxy-*sn*-glycero-3-phosphocholine (lyso-1-O-DPPC) generated were taken before adding PLA₂ and 1200 sec after the observed lag-time. 100 µl aliquots were withdrawn from the lipid suspension and rapidly mixed with 1 ml chloroform/methanol/acetic acid (2:4:1) solution in order to quench the enzymatic reaction. The solution was washed with 1 ml of water and 20 µl of the heavy organic phase was used for HPLC. The HPLC chromatograms in Fig. 3 show the amounts of 1-O-DPPC before and after (t = 2050 sec) the addition of PLA₂ (t = 800 sec) to the liposome suspension. HPLC analysis was made using a 5 µm diol column, a mobile phase composed of chloroform/methanol/water (730:230:30, v/v) and an evaporative light scattering detector. The turnover of the PLA₂ catalysed lipid hydrolysis of 1-O-DPPC to lyso-1-O-DPPC was measured by HPLC (see Table 1). The intrinsic enzyme fluorescence and 90° light scattering were measured as a function of time as shown in Fig. 2.

### Example 3

### Phospholipase A₂ induced release of an incapsulated water-soluble model drug

Multilamellar 1-O-DPPC-liposomes were made in the presence of fluorescent calcein in a self-quenching concentration of 20 mM by hydrating a film of 1-O-hexadecyl-2-hexadecanoyl-sn-glycero-3-phosphocholine in a HEPES buffer solution at pH=7.5 for one hour at 10°C above the phase transition temperature. Unilamellar liposomes were formed by extruding the multilamellar liposomes ten times through two stacked 100 nm polycarbonate filters. The unilamellar liposomes were rapidly cooled to a temperature below the transition temperature, and the calcein-containing 1-O-DPPC liposomes were separated from free calcein using a chromatographic column packed with Sephadex G-50.

Assay conditions for the PLA₂ induced calcein release were 25 µM unilamellar 1-O-DPPC-liposomes, 25 nM PLA₂, 150 mM KCL, 10 mM HEPES (pH 7.5 or 8.0), 1 mM NaN₃, 30 µM CaCl₂, and 10 µM EDTA. PLA₂ was added at time 900 sec to 2.5 ml of the thermostated 1-O-DPPC-liposome suspension equilibrated for at least 20 min at 37°C prior to addition of PLA₂. The percentage of calcein released is determined as: % Release = 100 x (I_{F(t}) - I_{B})/(I_{T} - I_{B}), where I_{F(t)} is the measured fluorescence at time t after addition of the enzyme, I_{B} is the background fluorescence, and I_{T} is the total fluorescence measured after addition of Triton X-100 which leads to complete release of calcein by breaking up the 1-O-DPPC-liposomes. PLA₂ induced at total release of 90 percent of the entrapped calcein in the 1-O-DPPC-liposomes as shown in Fig. 4.

### Example 4

### Phospholipase A₂ controlled permeability increase of a target model membrane

Multilamellar model membrane target liposomes were made in the presence of fluorescent calcein in a self-quenching concentration of 20 mM by hydrating a film of 1,2-O-dioctadecyl-*sn-*glycero-3-phosphatidylcholines (D-O-SPC) in a HEPES buffer solution at pH=7.5 for one hour at 10°C above the phase transition temperature (Tₘ=55°C). Unilamellar liposomes were made by extruding the multilamellar target liposomes ten times through two stacked 100 nm polycarbonate filters. The unilamellar liposomes were rapidly cooled to a temperature below the transition temperature, and the calcein-containing liposomes were separated from free calcein using a chromatographic column packed with Sephadex G-50. The unilamellar carrier liposomes composed of 1-O-hexadecyl-2-hexadecanoyl-sn-glycero-3-phosphocholine were prepared as described above. Calcein release from the target liposomes is determined by measuring the fluorescent intensity at 520 nm after excitation at 492 nm.

The concentrations of D-O-SPC and 1-O-DPPC-liposomes were 25 µM. Snake venom PLA₂ *(Agkistrodon piscivorus piscivorus)* was added (25 nM) to initiate the hydrolytic reaction leading to the formation of 1-O-hexadecyl-2-hydroxy-*sn*-glycero-3-phosphocholine (lyso-1-O-DPPC) and fatty acid hydrolysis products. As calcein is released from the D-O-SPC liposomes, due to the incorporation of the non-bilayer forming lyso-1-O-DPPC and fatty acid hydrolysis products into the target lipid membrane, a linear increase in the fluorescence at 520 nm after excitation at 492 nm is observed when calcein is diluted into the surrounding buffer medium as shown in Fig. 5. The percentage of calcein released is determined as described above (see Example 3).

### Example 5

### Hemolysis assay

Unilamellar fully hydrated liposomes with a narrow size distribution were prepared from 1-O-hexadecyl-2-hexadecanoyl-*sn*-glycero-3-phosphocholine (1-O-DPPC), and from 1-O-DPPC with 5 mol% 1-O-hexadecyl-2-hexadecanoyl-*sn*-glycero-3-phosphoethanolamine-N-[methoxy(polyethylene glycol)-350] (1-O-DPPE-PEG350) or with 5 mol%1-O-hexadecyl-2-hexadecanoyl-*sn*-glycero-3-phosphoethanolamine-N-[methoxy(polyethylene glycol)-2000 (1-O-DPPE-PEG2000) The lipids were hydrated in phosphate buffered saline (PBS). 1-O-octadecyl-2-O-methyl-*sn*-glycero-3-phosphocholine (ET-18-OCH₃) in PBS was included in the assay as a reference.

Hemolysis assay was performed as described by Perkins et al., *Biochim. et Biophys. Acta* **1327,** 61-68. Briefly, each sample was serially diluted with PBS, and 0.5 ml of each dilute suspension of 1-O-DPPC liposomes were mixed with 0.5 ml washed human red blood cells (RBC) [4% in PBS (v/v)]. For controls, 0.5 ml of the red blood cell suspension was mixed with either 0.5 ml buffer solution (negative hemolysis control) or 0.5 ml water (positive hemolysis control). Samples and standard were placed in a 37°C incubator and agitated for 20 hours. Tubes were centrifuged at low speed (2000 x G) for 10 minutes to form RBCs pellets. 200 µl of the supernatant was quantitated by absorbance at 550 nm using a Perkin-Elmer 320 scanning spectrophotometer. 100 percent hemolysis was defined as the maximum amount of hemolysis obtained from the detergent Triton X-100. The hemolysis profile in Fig. 6 shows a low hemolysis value (below 5 percent) for 2 mM 1-O-DPPC-liposomes. Figure 6 also shows that low concentrations of ET-18-OCH₃ induces a significant degree of hemolysis.

### Example 6

### Enhancement of phospholipase A2 activity by polymer grafted 1-O-DPPC lipids

Unilamellar fully hydrated liposomes with a narrow size distribution were prepared from 1-O-hexadecyl-2-hexadecanoyl-*sn*-glycero-3-phosphocholine (1-O-DPPC) and 1-O-DPPC with 5 or 10 mol% 1-O-hexadecyl-2-hexadecanoyl-*sn*-glycero-3-phosphoethanolamine-N-[methoxy(polyethylene glycol)-350] (1-O-DPPE-PEG350), as described in example 2. Assay conditions for the PLA₂ lag-time measurements were 0.15 mM unilamellar liposomes, 150 nM PLA₂, 150 mM KCL, 10 mM HEPES (pH 7.5), 1 mM NaN₃, 30 µM CaCl₂, and 10 µM EDTA. The catalytic reaction was initiated by adding 8.9 µL of a 42 µM PLA₂ stock solution to 2.5 ml of the thermostated liposomes suspension equilibrated for 800 seconds at 41 °C prior to addition of PLA₂. The time elapsed before the onset of rapid enzymatic activity is determined by a sudden increase in the intrinsic fluorescence from PLA₂ at 340 nm after excitation at 285 nm. The results shown in Fig. 7 show a significant decrease in the lag time when 5 and 10 mol% of 1-O-DPPE-PEG₃₅₀ is incorporated into the 1-O-DPPC liposomes.

### Example 7

### Preparation of micelles composed of 1-O-DPPE-PEG350, DSPE-PEG750/ DPPE-PEG750 and 1-O-DPPE-PEG2000.

Micelles were made from 1-O-hexadecyl-2-hexadecanoyl-*sn*-glycero-3-phosphoethanolamine-N-[methoxy(polyethylene glycol)-350] (1-O-DPPE-PEG350), di-octadecanoyl-*sn*-glycero-3-phosphoethanolamine-N-[methoxy(polyethylene glycol)-750 (DSPE-PEG750) or 1-O-hexadecyl-2-hexadecanoyl-*sn*-glycero-3-phosphoethanolamine-N-[methoxy(polyethylene glycol)-2000 (1-O-DPPE-PEG2000). Briefly, weighed amounts of the polymerised lipid were dissolved in chloroform. The solvent was removed by a gentle stream of N₂. The lipid films were then dried overnight under low pressure to remove trace amounts of solvent. Micelles were made by dispersing the dried polymerised lipids in a buffer solution containing: 150 mM KCL, 10 mM HEPES (pH = 7.5), 1 mM NaN₃, 30 µM CaCl₂ and 10 µM EDTA.

### Example 8

### Permeability increase of a target model membranes controlled by phospholipase A₂ hydrolysis of micelles

Multilamellar model membrane target liposomes were made in the presence of fluorescent calcein in a self-quenching concentration of 20 mM by hydrating a film of 1,2-O-dioctadecyl-*sn*-glycero-3-phosphatidylcholines (D-O-SPC) in a HEPES buffer solution at pH=7.5 for one hour at 10°C above the phase transition temperature (Tₘ=55°C). Unilamellar liposomes were made by extruding the multilamellar liposomes ten times through two stacked 100 nm polycarbonate filters. The unilamellar liposomes were rapidly cooled to a temperature below the transition temperature, and the calcein-containing liposomes were separated from free calcein using a chromatographic column packed with Sephadex G-50. Micelles composed of 1-O-hexadecyl-2-hexadecanoyl-*sn*-glycero-3-phosphoethanolamine-N-[methoxy(polyethylene glycol)-350] (1-O-DPPE-PEG350) dioctadecanoyl-*sn*-glycero-3-phosphoethanolamine-N-[methoxy(polyethylene glycol)-750 (DSPE-PEG750) or 1-O-hexadecyl-2-hexadecanoyl-*sn*-glycero-3-phosphoethanolamine-N-[methoxy(polyethylene glycol)-2000 (1-O-DPPE-PEG2000) were prepared as described in example 7. Calcein release from the target is determined by measuring the fluorescent intensity at 520 nm after excitation at 492 nm.

The concentrations of D-O-SPC and polymerised lipid micelles were 25 µM. Snake venom PLA₂ *(Agkistrodon piscivorus piscivorus)* was added (25 nM) to initiate the hydrolytic reaction leading to instant formation of the hydrolysis products polymerised lyso-1-O-DPPE and the corresponding free fatty acid. As calcein is released from the D-O-SPC liposomes, due to the incorporation of the non-bilayer forming polymerised lyso-1-O-DPPE and fatty acid into the target lipid membrane, a linear increase in the fluorescence at 520 nm after excitation at 492 nm is observed when calcein is diluted into the surrounding buffer medium as shown in Fig. 8. The percentage of calcein released is determined as described in example 3. PLA₂ catalysed hydrolysis of 1-O-DPPE-PEG350 induced the fastest release rate, whereas the DPPE with the longest polymer chain (PEG2000) attached to the head group induced the slowest rate of release.

### Example 9

### Hydrolysis of micelles composed of DSPE-PEG750

The hydrolysis of micelles composed DSPE-PEG750 was followed by analysis of the amount of stearic acid generated. The catalytic reaction was initiated by adding 8.9 µL of a 42 µM PLA₂ (150 nM) stock solution to 2.5 ml a thermostated micelle solution of DSPE-PEG750 (0.150 mM) equilibrated at 45 °C for 600 seconds prior to addition of PLA₂. The characteristic lag-burst behaviour of PLA₂ towards the micelles is signalled by a sudden increase in the intrinsic fluorescence from PLA₂ at 340 nm after excitation at 285 nm followed by a concomitant decrease in the 90° light scattering from the lipid suspension (Hønger et al., *Biochemistry* **35**, 9003-9006). Samples for HPLC analysis of the amount of stearic acid generated were taken before adding PLA₂ and 100 sec after the observed lag-time. The HPLC chromatograms in Fig. 9 shows the amount of stearic acid generated 100 sec after the observed lag time (10 sec) at 45°C. The amount (0.156 mM) of stearic acid generated by hydrolysis was equal to 100% hydrolysis of the DSPE-PEG750 polymer-lipids. HPLC analysis was made using a 5 µm diol column, a mobile phase composed of chloroform/methanol/water (730:230:30, v/v) and an evaporative light scattering detector (see example 2).

### Example 10

### Model examples

Polymer-coated liposomes can act as versatile drug-delivery systems due to long vascular circulation time and passive targeting by leaky blood vessels in diseased tissue. In the examples herein are described an experimental model system illustrating a new principle for improved and programmable drug-delivery which takes advantage of an elevated activity of extracelluar phospholipase A₂ at the diseased target tissue. The phospholipase A₂ hydrolyses a lipid-based proenhancer in the carrier liposome, producing lysophospholipid and free fatty acid, which are shown in a synergistic way to lead to enhanced liposome destabilisation and drug release at the same time as the permeability of the target membrane is enhanced. The proposed system can be made thermosensitive and offers a rational way for developing smart liposome-based drug delivery systems by incorporating into the carrier specific lipid-based proenhancers, prodestabilisers or prodrugs that automatically become activated by phospholipase A₂ only at the diseased target sites, such as inflammed or cancerous tissue.

Drug-delivery systems based on liposomal carriers in the 100 nm range are one of the modern microcarrier therapeutic systems that hold a promise for coming close to realising Paul Erlich's early vision of a "magic bullet" for treatment of diseases. Liposomes made of biocompatible, non-toxic phospholipids provide a system for efficient formulation and encapsulation of toxic drugs which effectively can evade the immune system.

The drug assumes the altered pharmacokinetics of the liposomal carrier and can in principle be targeted to the diseased tissue by using a combination of physico-chemical and pathophysiological factors at the sites of the liposome carrier and the target membrane, respectively. Liposomes incorporated with glycolipids or lipopolymers, such as polyethylene-glycol (PEG)-lipids, known as liposomes, display an improved stability in the vascular system, possibly due to steric protection caused by the polymer coating. The prolonged circulation time of these liposomes combined with increased vascular porosity of diseased tissue, have formed the basis for positive clinical results for specific systems, including anticancer drugs like doxorubicin as well as antibacterial and anti-inflammatory drugs.

Liposomes are self-assembled lipid systems and their stability is therefore to a large extent controlled by non-specific physical interactions. Insight into the molecular control of the physical properties of liposomes is therefore important for manipulating and tailoring the liposomal properties in relation to specific drug-delivery purposes. As an example, the thermally induced gel-fluid lipid phase transition has been exploited and optimised design systems for enhanced release of drugs due to hyperthermia. Recently, programmable fusogenic PEG-liposomes containing the anticancer drug mitoxantrone have been constructed using a time-delayed release of bilayer-stabilising lipids of the liposomes which are accumulated at the tumour sites by extravasation. It would be desirable if an intelligent and versatile drug-delivery system could be designed which has built in a dual virtual trigger mechanism of simultaneous (i) enhanced drug release selectively at the target tissue and (ii) enhanced transport of the drug into the diseased cells. This principle is illustrated schematically in Fig 11.a.

By the examples herein is described the development of a simple and operative experimental biophysical model system which sustains such a dual mechanism to be triggered at the pathological target sites. The model assumes elevated activity of extracellular phospholipase A₂ at the diseased sites as is the case in inflammed and cancerous tissue where the level of extracellular PLA₂ can be manifold magnified. Upon exposure to extracellular PLA₂, the phospholipids of the PEG-liposomes have been shown to suffer enhanced hydrolysis compared to conventional bare liposomes. This leads to destabilisation of the liposome and enhanced release of the encapsulated drug. The hydrolysis products, lyso-phospholipids and free fatty acids, act in turn as absorption enhancers for drug permeation across the target membrane. In this way the phospholipids of the carrier liposome behave as prodestabilisers at the site of the carrier and as proenhancers at the site of the target membrane. Molecular details of this principle are illustrated schematically in Fig. 11.b.

The experimental model system consists of a polymer-coated liposome carrier and a model target membrane. The carrier is a 100 nm unilamellar liposome made of dipalmitoyl phosphatidylcholine lipids (DPPC) with 2.5 mol% lipopolymer of the type dipalmitoyl phosphatidylethanolamine (DPPE)-PEG₂₀₀₀. The target membrane is another liposome made of 1,2-O-dioctadecyl-sn-glycero-phosphatidylcholine (D-O-SPC) which is a phospholipid where the acyl linkages of the stearoyl chains are ether bonds. In contrast to DPPC, D-O-SPC is inert towards PLA₂-catalysed hydrolysis thereby mimicking the stability of an intact target cell membrane toward degradation by its own enzymes. This experimental assay, which permits simultaneous as well as separate investigation of the effect of destabilisers at the carrier liposomes and the effect of enhancers at the target membrane, involves entrapment of a water-soluble fluorescent calcein model drug in a self-quenching concentration, in the interior of the non-hydrolysable target liposome, rather than in the carrier liposome. The enhanced level of extracellular PLA₂ at the target membrane can then be simulated by adding extracellular PLA₂ to initiate the hydrolytic reaction in a suspension of the carrier and target liposomes. The permeation of calcein across the D-O-SPC target membrane is subsequently monitored by the increase in fluorescence. In order to investigate the effect of the presence of the PEG-lipids in the carrier liposome, a similar experiment was performed with conventional bare DPPC liposomes. Furthermore, in order to compare and discriminate the permeability enhancing effect of lyso-phospholipids from that of free fatty acids, experiments without enzymes were carried out where lyso-phospholipids and free fatty acids were added simultaneously or separately to the target liposomes.

In Fig. 12.a are shown the results for the release of calcein as a function of time after adding PLA₂ to the system. The reaction time-course of the particular PLA₂ used has a characteristic lag-burst behaviour with a so-called lag time which conveniently can be used as a measure of the enzymatic activity. A dramatic decrease in the lag time and a concomitant enhancement of the rate of release are observed when the carrier liposomes contain the lipopolymers, DPPE-PEG₂₀₀₀, in accordance with previous findings of enhanced extracellular PLA₂ degradation of polymer-coated liposomes.

These results suggest that the products of the PLA₂-catalysed hydrolysis of the DPPC lipids of the carrier, lyso-phospholipid and free fatty acid, which are produced in a 1:1 mixture, are incorporated into the target membrane, leading to a large increase in membrane permeability. These products, which have very low water solubility, are known, due to their non-cylindrical molecular shapes, to induce a curvature stress field in the membrane or small-scale lateral phase separation which induce membrane defects and increased permeability. This is substantiated by the data in Fig. 13 which show that the addition of lyso-phospholipid or fatty acid separately to the present target system, in the absence of PLA₂, leads to an increased rate of calcein release across the target membrane. However, the crucial finding is that if lyso-phospholipid and free fatty acid are added simultaneously in a 1:1 mixture, a dramatic enhancement in the rate of release is observed as shown in Fig. 13. This strongly suggests that the two enhancers act in a synergistic fashion, thereby highlighting the unique possibility in exploiting PLA₂-catalysed hydrolysis for combined destabilisation of the carrier liposome and enhancement of drug transport across the target membrane. The synergistic effect is further augmented by the fact that extracellular PLA₂ is activated by its own hydrolysis products revealing the degradable phospholipids of the carrier liposome as a kind of proactivators.

It should be pointed out that the effect in the present drug-delivery model system of using lipids as proenhancers and prodestabilisers via extracellular PLA₂ activity is dynamic and refers to an intrinsic time scale. This time scale is the effective retention time of the carrier liposomes near the target membrane. The more rapidly the enzyme becomes active, the faster is the drug release and the larger the drug absorption during the time which the carrier spends near the target. Furthermore, the faster the enzyme works the more readily it becomes available for hydrolysis of other drug-carrying liposomes that approach the diseased target site. Once it has been established that extracellular PLA₂ activity can be used to control drug release, several rational ways open up for intelligent improvements of the proposed drug-delivery system via use of well-known mechanisms of altering extracellular PLA₂ activity by manipulating the physical properties of the lipid bilayer to which the enzyme is known to be sensitive. Hence the strategy is to modify certain physical properties of the carrier liposomes without significantly changing their vascular circulation time. We shall illustrate this general principle by demonstrating the effects of both a physico-chemical factor, the lipid composition of the carrier, and an enviromental (thermodynamic) factor, the local temperature at the target site.

Short-chain phospholipids, such as didecanoyl phosphatidylcholine (DCPC), activate extracellular PLA₂. The effect on calcein permeation across the target membranes induced by incorporation of a small amount of DCPC into the carrier PEG-liposomes is also shown in Fig. 12.a. The release is very fast due to an almost instantaneous activation of the enzyme. We have furthermore found that extracellular PLA₂ becomes deactivated (data not shown) when a large amount of cholesterol (≈20 mol %) is incorporated into liposomes. In contrast we find that a small amount of cholesterol (≈3 mol%) activates extracellular PLA₂. These significant findings are of particular interest since the blood circulation time of PEG-liposomes has been reported to be almost the same without cholesterol as with large amounts of cholesterol.

Temperature is known to have a dramatic and highly non-linear effect on extracellular PLA₂ activation in the region of the gel-fluid phase transition of saturated phospholipid bilayers. This effect is not caused by changes in the enzyme but by dramatic lateral structural changes in the lipid bilayer. It is possible to take advantage of this effect in the present drug-delivery system as suggested by the data in Fig. 12.b. As the temperature approaches the transition temperature at 41°C, the rate of calcein release is progressively enhanced as quantified by the time of 50% calcein release, t_{50%}, shown in the insert to Fig. 12.b. It has previously been suggested that hypertermia could be exploited to enhance drug release, and that local heating at predefined tumour areas could be used to locally destabilise drug-carrying liposomes, by exploiting the enhanced leakiness of liposomes at their phase transition. In the new model drug-delivery system proposed here, these thermosensitive possibilities are integrated and fully exploited via the thermal sensitivity of extracellular PLA₂ to the physical properties of the carrier liposome. In contrast to the case where the thermic effect can only be achieved by a local temperature increase using external heating sources at a predetermined tumour site of some minimal size, the PLA₂-controlled release will be enhanced everywhere where temperature and extracellular PLA₂ concentration are elevated, e.g. in inflammed tissue, independent of the size of the diseased region and without requiring a preceding localisation of the diseased tissue.

DPPC, DCPC, D-O-SPC, and DPPE-PEG₂₀₀₀ were obtained from Avanti Polar Lipids. The DPPE-PEG₂₀₀₀ lipopolymer contains 45 monomers in the PEG polymer chain. Purified snake venom PLA₂ *(Agkistrodon piscivorus piscivorus)* was a generous gift from dr. R. L. Biltonen. This PLA₂ enzyme belongs to the class of low-molecular weight, 14kD secretory enzymes which display structural similarity to human extracellular phospholipase A₂. Multilamellar target liposomes in the presence of fluorescent calcein in a self-quenching concentration of 20mM were made by hydrating a film of D-O-SPC in a HEPES buffer solution at pH=7.5 for one hour at 10°C above the phase transition temperature Tₘ=55°C. Unilamellar liposomes were made by extruding the multilamellar liposomes ten times through two stacked 100nm polycarbonate filters. The unilamellar liposomes were rapidly cooled to a temperature below the transition temperature, and the calcein-containing liposomes were separated from free calcein using a chromatographic column packed with Sephadex G-50. The unilamellar carrier liposomes of DPPC, DCPC and DPPE-PEG₂₀₀₀ were prepared in a similar fashion Tₘ=41°C). Calcein release from the target liposomes is determined by measuring the fluorescent intensity at 520nm after excitation at 492nm. All measurements are performed at temperatures where the lipids of both the carrier and target liposomes are in the gel state.

### Example 11

### Phospholipase A₂ concentration dependent release assay

Multilamellar 1-O-DPPC-liposomes with 10 mol% 1-O-DPPE-PEG350 were made in the presence of fluorescent calcein in a self-quenching concentration of 20 mM by hydrating a film of 90% 1-O-hexadecyl-2-hexadecanoyl-*sn*-glycero-3-phosphocholine and 10% 1-O-hexadecyl-2-hexadecanoyl-*sn*-glycero-3-phosphoethanolamine-N-[methoxy(polyethylene glycol)-350] in a HEPES buffer solution at pH=7.5 for one hour at 10 °C above the phase transition temperature. Unilamellar liposomes were formed by extruding the multilamellar liposomes ten times through two stacked 100 nm polycarbonate filters. The unilamellar liposomes were rapidly cooled to a temperature below the transition temperature, and the calcein-containing liposomes were separated from free calcein using a chromatographic column packed with Sephadex G-50.

Assay conditions for the PLA₂ induced calcein release were 25 µM unilamellar liposomes, 50, 1 and 0.02 nM PLA₂, 150 mM KCL, 10 mM HEPES (pH 7.5), 1 mM NaN₃, 30 µM CaCl₂, and 10 µM EDTA. PLA₂ was added to 2.5 ml of the thermostated micelle solution equilibrated for at least 300 sec at 35.5°C prior to addition of PLA₂. The percentage of calcein released is determined as: % Release = 100 x (I_{F(t)} - I_{B})/(I_{T} - I_{B}), where I_{F(t)} is the measured fluorescence at time t after addition of the enzyme, I_{B} is the background fluorescence, and I_{T} is the total fluorescence measured after addition of Triton X-1 00 which leads to complete release of calcein by breaking up the 1-O-DPPC-liposomes. Figure 14 show that the induced release of calcein was slowest when only 0.02 nM PLA₂ was added to the liposome suspension.

### Example 12

### Fluorescence measurements of extracellular PLA₂ activity in MCF-7 and Lewis Lung cancer cell lines.

**Cell culturing:** The cell lines are cultured in RPMI-1640 medium supplemented with 10% fetal calf serum in 5% carbon dioxide. The cell lines are grown in 20 ml of the medium in culture flask (navn og type). Culture samples are collected when the cell density reaches a confluent density of 1 x 10⁷ cells and stored at -80°C until assayed. The culture samples are centrifuged at 2000 x G for 10 min and the extracellular PLA₂ activity in the supernatants is measured by fluorescence techniques. As described in example 3 and 11.

### Example 13

### Growth inhibition assay

Growth inhibition assay will be performed to determine the Gl₅₀ parameter (the concentration of drug which inhibits cell growth 50%) using a sulforhodamine B (SRB) assay as described by Peters et al. in Lipids 32, (1997). Cell lines expressing extracellular phospholipase A₂ are grown as described in example 12 in medium PRMI 1640 with L-glutamine supplemented with 10% fetal bovine serum. 100 µl of the cells are transferred to 96-well microtiter plates and incubated for 24 hours at 37 °C., 100% humidity, and 5% CO₂. Medium (100 µL) is added to designated "time zero" plates, which are fixed with 50 µL of 50% trichloroacetic acid (wt/vol) or 80% trichloroacetic acid (suspension cells). The supernatant is then discarded and the plates are rinsed with water and air-dried. 1-O-DPPC-liposome 1-O-DPPC-liposomes incorporated with 10% 1-O-DPPE-PEG350 and ET-18-OCH3 are added to the non-fixed plates at twice the predetermined highest concentration and serial diluted across the plates. Growth control wells receive 100 µL of the medium. The cells are incubated for 72 hours under the above conditions. The treated plates are acid-fixed, rinsed and dried as above. 100 µL of 0.4% SRB in 1 % acetic acid is added to the plates and incubated at room temperature for 10 min. Unbound stain is removed by rinsing the plates with 1% acetic acid. The plates are air-dried and the bound strain is solubilised with 100µL of 10 mM Tris buffer, and the optical density is read spectrophotometrically at 490 nm. Percentage growth is calculated as: (T-T₀)/(C-T₀) x 100, where T = mean optical density of treated wells at a given drug concentration, T₀ = mean optical density of time zero wells, and C = mean optical density of control wells. If T<T₀, which means that cell death has occurred, then percentage cell death is calculated as (T-T₀)/(T₀) x 100. By varying the drug concentration, dose-response curves can be generated, and Gl₅₀ values can be calculated.

### Example 14

### Animal Studies

A maximum tolerated dose (MTD) study will be performed as described in Ahmad et al., Cancer Res. 57 (1997). Groups of female C57/BL6 mice (5/group; weight, 18-22 g) are injected with various doses of 1-O-DPPC-liposomes (25-600 mg/kg) , 1-O-DPPC-liposomes incorporated with 10% 1-O-DPPE-PEG350 (25 - 600 mg/kg) and ET-18-OCH3 (12.5-100 mg/kg) in PBS.

For multiple dose studies 25- 300 mg/kg 1-O-DPPC-liposomes, 25-300 mg/kg 1-O-DPPC-liposomes incorporated with 10% 1-O-DPPE-PEG350 and 12.5 -75 mg/kg ET-18-OCH3 were administered i.v. for five consecutive days. The mice are weighed three times weekly, and mortality is recorded on a daily basis. Experiments are terminated 1 month after the initial treatment and MTD is determined.

*In vivo* anti-cancer studies will be performed in immunodeficient nude mice (eg NMRI nu/nu female mice) inoculated in with tumour cell lines selected based on the results in previous *in vitro* experiments. Based on literature studies (Ahmad I. et al., Cancer Research 1997; 57: 1915-1921), relevant cell lines could potentially include Lewis Lung Cancer (LLC), B16/F10 melanoma and P388 Leukemia cells.

Each experiment will include between 30-60 mice, kept in Scantainers (or similar) under semi-sterile conditions. Following inoculation, tumours will be left to grow for 4-12 days (depending on the tumour type) or until measurable. Animals will then be divided into groups of 10 by a procedure ensuring that the tumour load is comparable between groups. Animals will receive treatment with either the lysoetherlipid, etherlipid or a negative control for a period of 3-8 weeks, depending on the tumour type studied. Additional groups of animals may be added in order to study different doses of lysoetherlipid and etherlipid, respectively. All medications will be administered according to a dosing regimen decided based on previous pharmacokinetic experiments by the i.v. or i.p. route.

Assessment of the anti-cancer effect may include counting of primary tumours (as for the B16/F10 and LLC models), measurement of tumour size in 2 perpendicular angles (for subcutaneous solid tumours in case such models are employed) and assessment of survival (as for the P388 model).

Anticancer effect experiments also include the use of Lewis lung cancer (LLC), which will be injected i.m. This rapidly growing tumor metastasizes from the i.m. tumors to the lungs and experiments will be done in order to determine the effects of lipid derivative liposomes, etherlipid, and ET-18-OCH3 on spontaneous lung metastasis development.

Body weight (as a measurement of toxicity) will be recorded before, during and at the end of the experiment. In addition, mice will be observed on a day by day basis for any clinical adverse reactions.

At the end of treatment, blood will be collected by cardiac puncture to study the degree of hemolysis.

Depending on the type of data recorded, suitable statistical methods will be used to compare the groups.

Pharmacokinetic studies using e.g. radiolabled lipid derivative liposomes will be carried out using healthy animals and animals injected with different tumor types expressing extracellular phospholipase A2 in high amounts, e.g. breast cancer, genital tumors, gastric tumors (Yamashita et al. Br. J. Cancer (1994) 6, 1166; Kallajoki et al. Prostate (1998) 35, 263; Yamashita et al. Biochem. Biophys. Res. Commun. (1994) 198, 878; Abe et al. Inj. J. Cancer (1997) 74, 245). The blood circulation time and tissue distribution of PEGylated lipid derivative liposomes will be determined. Also the ability of the long circulating lipid derivative liposomes to extravasate through the leaky capillaries and accumulate in the tumors where lipid derivative are turning into ether lipids by means of extracellular phospholipase A2 catalysed hydrolysis will be investigated using, e.g. (double)radioactive labelled lipid derivatives.

### Example 15

### Clinical studies

Early clinical studies will be performed in patients with advanced cancer (cancer type to be determined based on results obtained in *in vivo* animal studies). The "Notes for Guidance in Evaluation of Anticancer Medicinal Products in Man, The European Agency for Evaluation of Medicinal Products (EMEA), 1996 or subsequent updates will be adhered to. The first study will evaluate the safety, efficacy and pharmacokinetic parameters by single dose as well as repeated dose administration. A modified Fibronacci regimen will be used (Simon R. et al., Journal of the National Cancer Institute 1997; 89: 1138-1147). Consideration will be given to selection of eligible patients based on previous immunohistochemical detection of high mPLA-2 levels in tumour specimens, if possible.

### Example 16

### In vivo hemolysis

### Assay

Groups of mice (n = 5/group; weight 18-22 g) were treated i.v. with buffer (PBS), ET-18-OCH₃ (50 mg/kg), and liposomes composed of 1-O-DPPC with 5 mol% 1-O-DPPE-PEG2000 (68.7 and 137.5 mg/kg). 30 min after injection, 100 µl blood was collected into heparinised tubes directly from decapitated, CO₂-anesthetised mice. The blood was centrifuged at 500 x G for 10 min, and plasma was removed and stored at -20°C until analysed.

The degree of hemolysis was measured by absorbance at 550 nm using a Perkin-Elmer 320 scanning spectrophotometer. 25 µl plasma was mixed with 2,5 ml PBS or 2.5 ml 10% triton X-1 00. 100 percent hemolysis was defined as the maximum amount of hemolysis obtained from the plasma of PBS treated mice mixed with 10% Triton X-100 and 0 percent as plasma from PBS treated mice mixed with PBS.
The hemolysis profile in table 2 shows a low hemolysis value for the mice treated with 2 mM liposomes composed of 1-O-DPPC with 5 mol% 1-O-DPPE-PEG2000. The mice treated with ET-18-OCH₃ died within the 30 minutes period.

**Table 2.**

| Percent hemolysis, ± standard error, after 30 min and the number of surviving mice in each group. | | | | |
|---|---|---|---|---|
| Treatment | PBS | ET-18-OCH₃ | 1-O-DPPC with 5 mol% 1-O-DPPE-PEG2000 | |
| | | 50 mg/kg | 68.7 mg/kg | 137.5 mg/kg |
| Hemolysis | 1.7 % (± 0.2) | ND (died) | 4.0 % (± 2.4) | 6.6 % (± 0.7) |
| Survival (30 min) | 3/3 | 0/5 | 5/5 | 5/5 |

## Claims

1. A lipid based drug delivery system for administration of a drug substance, wherein the drug substance is incorporated in the system, said system including lipid derivatives which has (a) an aliphatic group of a length of at least 7 carbon atoms and an organic radical having at least 7 carbon atoms, and (b) a hydrophilic moiety, where the lipid derivative furthermore is a substrate for extracellular phospholipase A2 to the extent that the organic radical can be hydrolytically cleaved off, said system having included therein lipopolymers or glycolipids so as to present hydrophilic chains on the surface of the system.

2. The lipid based drug delivery system according to claim 1, wherein the aliphatic group remains substantially unaffected, so as to result in an organic acid fragment or an organic alcohol fragment and a lysolipid fragment.

3. A drug delivery system according to claim 1, wherein the lipopolymers or glycolipids are represented by a fraction of the lipid derivative.

4. A drug delivery system according to any of the claims 1-3, wherein the polymer of the lipopolymer is selected from polyethylene glycol, poly(lactic acid), poly(glycolic acid), poly(lactic acid)-poly(glycolic acid) copolymers, polyvinyl alcohol, polyvinylpyrrolidone, polymethoxazoline, polyethyloxazoline, polyhydroxypropyl methacrylamide, polymethacrylamide, polydimethylacrylamide, and derivatised celluloses.

5. A drug delivery system according to any of the claims 1-4, wherein the organic radical which can be hydrolytically cleaved off, is an auxiliary drug substance or an efficiency modifier for the drug substance.

6. A drug delivery system according to any of the claims 1-5, wherein the lipid derivative is a lipid derivative of the following formula: 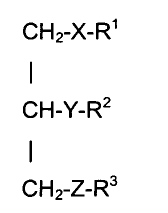 wherein
X and Z independently are selected from O, CH₂, NH, NMe, S, S(O), and S(O)₂;
Y is -OC(O)-, Y then being connected to R² via either the oxygen or carbonyl carbon atom;
R¹ is an aliphatic group of the formula Y¹Y²;
R² is an organic radical having at least 7 carbon atoms;
where Y¹ is -(CH₂)ₙ₁-(CH=CH)ₙ₂-(CH₂)ₙ₃-(CH=CH)ₙ₄-(CH₂)ₙ₅-(CH=CH)ₙ₆-(CH₂)ₙ₇-(CH=CH)ₙ₈ᵣ-(CH₂)ₙ₉, and the sum of n1+2n2+n3+2n4+n5+2n6+n7+2n8+n9 is an integer of from 9 to 29; n1 is zero or an integer of from 1 to 29, n3 is zero or an integer of from 1 to 20, n5 is zero or an integer of from 1 to 17, n7 is zero or an integer of from 1 to 14, and n9 is zero or an integer of from 1 to 11; and each of n2, n4, n6 and n8 is independently zero or 1; and Y² is CH₃ or CO₂H; where each Y¹-Y² independently may be substituted with halogen or C₁₋₄-alkyl,
R³ is selected from phosphatidic acid (PO₂-OH), derivatives of phosphatidic acid and bioisosters to phosphatic acid and derivatives thereof.

7. A drug delivery system according to claim 6, wherein R² is an aliphatic group of a length of at least 7 carbon atoms.

8. A drug delivery system according to claim 7, wherein R² is a group of the formula Y¹Y².

9. A drug delivery system according to any of the claims 1-8, wherein at least a fraction of the prodrug is of the formula defined in claim 7, wherein R³ is a derivative of phosphatidic acid to which a polymer selected from polyethylene glycol, poly(lactic acid), poly(glycolic acid), poly(lactic acid)-poly(glycolic acid) copolymers, polyvinyl alcohol, polyvinylpyrrolidone, polymethoxazoline, polyethyloxazoline, polyhydroxypropyl methacrylamide, polymethacrylamide, polydimethylacrylamide, and derivatised celluloses, is covalently attached.

10. A drug delivery system according to any of the claims 1-9, wherein the lipid derivative constitutes 15-100 mol% of the total dehydrated system.

11. A drug delivery system according to any of the claims 1-10, wherein the lipopolymer constitutes 2-50 mol% of the total dehydrated system.

12. A drug delivery system according to any of the claims 1-11, wherein the system is in the form of liposomes.

13. A drug delivery system according to any of the claims 1-12, wherein the second drug substance is a therapeutically and/or prophylactically active substance selected from (i) antitumor agents, (ii) antibiotics and antifungals, and (iii) antiinflammatory agents.

14. A pharmaceutical composition comprising the drug delivery system according to any of the claims 1-13 and optionally a pharmaceutically acceptable carrier.

15. The drug delivery system according to any of the claims 1-13 for use as a medicament.

16. The use of a drug delivery system according to any of the claims 1-13 for the preparation of a medicament for the treatment of diseases or conditions associated with a localised increase in extracellular phospholipase A2 activity in mammalian tissue.

17. The use according to claim 16, wherein the diseases or conditions are selected from the group consisting of inflammatory conditions and cancer.

18. The use according to claim 17, wherein the type of cancer is selected from the group consisting of brain cancer, breast cancer, lung cancer, colon cancer, ovarian cancer, leukemia, lymphoma, sarcoma and carcinoma.

19. The use according to any of claims 17-18, wherein the increase in extracellular phospholipase A2 activity is a least 25% compared to the normal level of activity in the tissue in question.

20. The lipid based drug delivery system according to any of the claims 1-5, wherein the lipid derivative is a lipid derivative of the following formula: 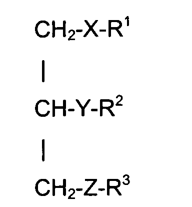 wherein
X=Y;
Y is -OC(O)-, Y then being connected to R² via either the oxygen or carbonyl carbon atom;
Z is selected from O, CH₂, NH, NMe, S, S(O), and S(O)₂;
R¹ is an aliphatic group of the formula Y¹Y²;
R² is an organic radical having at least 7 carbon atoms;
where Y¹ is -(CH₂)ₙ₁-(CH=CH)ₙ₂-(CH₂)ₙ₃-(CH=CH)ₙ₄-(CH₂)ₙ₅-(CH=CH)ₙ₆-(CH₂)ₙ₇-(CH=CH)ₙ₈ᵣ-(CH₂)ₙ₉, and the sum of n1+2n2+n3+2n4+n5+2n6+n7+2n8+n9 is an integer of from 9 to 29; n1 is zero or an integer of from 1 to 29, n3 is zero or an integer of from 1 to 20, n5 is zero or an integer of from 1 to 17, n7 is zero or an integer of from 1 to 14, and n9 is zero or an integer of from 1 to 11; and each of n2, n4, n6 and n8 is independently zero or 1; and Y² is CH₃ or CO₂H; where each Y¹-Y² independently may be substituted with halogen or C₁₋₄-alkyl,
R³ is selected from phosphatidic acid (PO₂-OH), derivatives of phosphatidic acid and bioisosters to phosphatic acid and derivatives thereof.

21. A drug delivery system according to claim 20, wherein R² is an aliphatic group of a length of at least 7 carbon atoms.

22. A drug delivery system according to claim 7, wherein R² is a group of the formula Y¹Y².

23. The lipid based drug delivery system according to claims 1 and 22, wherein the system is in the form of liposomes.

24. The lipid based drug delivery system according to any of the claims 1-5, 20-23, wherein the drug substance is a therapeutically and/or prophylactically active substance selected from (i) antitumor agents, (ii) antibiotics and antifungals, and (iii) antiinflammatory agents.

25. A pharmaceutical composition comprising the drug delivery system according to any of the claims 1-5, 20-24 and optionally a pharmaceutically acceptable carrier.

26. The drug delivery system according to any of the claims 1-5, 20-24 for use as a medicament.

27. The use of a drug delivery system according to any of the claims 1-5, 20-24 for the preparation of a medicament for the treatment of diseases or conditions associated with a localised increase in extracellular phospholipase A2 activity in mammalian tissue.

28. The use according to claim 27, wherein the diseases or conditions are selected from the group consisting of inflammatory conditions and cancer.

29. The use according to claim 28, wherein the type of cancer is selected from the group consisting of brain cancer, breast cancer, lung cancer, colon cancer, ovarian cancer, leukemia, lymphoma, sarcoma and carcinoma.

30. The use according to any of claims 27-28, wherein the increase in extracellular phospholipase A2 activity is a least 25% compared to the normal level of activity in the tissue in question.
